# EUROPEAN PATENT SPECIFICATION

(11) **EP 4 422 478 B1**
(45) Date of publication and mention of the grant of the patent: **12.08.2026**
(21) Application number: 22886308.0
(22) Date of filing: 25.10.2022
(51) Int. Cl.: A61B 5/00, A61B 5/1473, A61B 5/145, A61B 10/00, G01N 27/327, G01N 27/414

(54) **WEARABLE MICRONEEDLES-BASED EXTENDED GATE FIELD-EFFECT TRANSISTOR FOR REAL-TIME DETECTION OF BIOMARKERS FROM INTERSTITIAL FLUID**
TRAGBARER MIKRONADELBASIERTER FELDEFFEKTTRANSISTOR MIT ERWEITERTEM GATE ZUR ECHTZEITDETEKTION VON BIOMARKERN AUS INTERSTITIELLER FLÜSSIGKEIT
TRANSISTOR À EFFET DE CHAMP PORTABLE À GRILLE ÉTENDUE BASÉ SUR DES MICRO-AIGUILLES POUR DÉTECTION EN TEMPS RÉEL DE BIOMARQUEURS DANS UN FLUIDE INTERSTITIEL

(30) Priority: 25.10.2021 IL 28757721
(43) Date of publication of application: 04.09.2024
(73) Proprietor: TECHNION RESEARCH & DEVELOPMENT FOUNDATION LIMITED, 3200004 Haifa (IL)
(72) Inventor: HAICK, Hossam, 3200004 Haifa (IL); ZHENG, Youbin, 3200004 Haifa (IL); OMAR, Rawan, 3200004 Haifa (IL); SALIBA, Walaa, 3200004 Haifa (IL)
(74) Representative: Straus, Alexander
(86) International application number: PCT/IL2022/051120
(87) International publication number: WO 2023/073691

(56) References cited:
- WO-A1-2019/044993
- WO-A1-2019/044993
- WO-A1-2021/118431
- US-A1- 2018 338 713
- US-A1- 2021 321 942
- MINAMI TSUYOSHI ET AL: "An extended-gate type organic field effect transistor functionalised by phenylboronic acid for saccharide detection in water", CHEMICAL COMMUNICATIONS, vol. 50, no. 98, 1 January 2014 (2014-01-01) - 27 March 2025 (2025-03-27), UK, pages 15613 - 15615, XP093263391, ISSN: 1359-7345, DOI: 10.1039/C4CC07498J
- KOKLU ANIL ET AL: "Organic Bioelectronic Devices for Metabolite Sensing", vol. 122, no. 4, 5 October 2021 (2021-10-05), US, pages 4581 - 4635, XP093148693, ISSN: 0009-2665, Retrieved from the Internet <URL:https://pubs.acs.org/doi/pdf/10.1021/acs.chemrev.1c00395> [retrieved on 20250327], DOI: 10.1021/acs.chemrev.1c00395
- HALIMA HAMDI BEN, ERRACHID ABDELHAMID, JAFFREZIC‐RENAULT NICOLE: "Electrochemical Affinity Sensors Using Field Effect Transducer Devices for Chemical Analysis", ELECTROANALYSIS, vol. 35, no. 1, 1 January 2023 (2023-01-01), US , XP093061973, ISSN: 1040-0397, DOI: 10.1002/elan.202100451
- TOMAS GUINOVART, GABRIELA VALDES-RAMIREZ, JOSHUA R. WINDMILLER, FRANCISCO J. ANDRADE, JOSEPH WANG: "Bandage-Based Wearable Potentiometric Sensor for Monitoring Wound pH", ELECTROANALYSIS, vol. 26, no. 6, 1 June 2014 (2014-06-01), US , pages 1345 - 1353, XP055673855, ISSN: 1040-0397, DOI: 10.1002/elan.201300558

## Description

### FIELD OF THE DISCLOSURE

The present disclosure is directed to a wearable skin-mountable extended gate field effect transistor (EGFET) device for detecting and/or measuring a constituent of an interstitial fluid from the skin of a subject.

### BACKGROUND OF THE DISCLOSURE

Smart wearable devices based on sweat or interstitial fluids (ISF) biosensing have gained considerable advantage in monitoring multi-physiological indexes and biomarkers, such as minerals, trace elements, lactic acid, urea, and volatile organic compounds, especially in view of the growing demand for convenient remote healthcare monitoring and telemedicine.

Currently available systems for analyzing body fluids biomarkers rely on collecting a sample followed by an elaborate offline analysis, which often requires the use of bulky laboratory devices. These systems do not allow continuous measurements and continuous monitoring of fluid composition, which are of supreme importance for the early detection of chronic diseases and play a crucial role in all treatment and prevention strategies.

One of the most common methods to measure biomarkers levels is by taking a blood sample, which is not only painful to collect, but is also time-consuming procedure. More importantly, this method requires trained personnel and does not provide continuous measurements. Compared to blood, ISF sampling is easier, painless, and enables safer online and *in-situ* biomarker analysis for long-term monitoring, as well as eliminating the need for extraction methods or dealing with blood clots. Compared to sweat biosensors that require tedious extraction methods and are not time-efficient, ISF could provide biomarker analysis without the need for excessive sweating exercises or being affected by confounding factors linked with the sweat conditions, such as poor sweat rates, sample evaporation, sample freshness, and/or contamination from the skin. As for urine, although easily accessible, it cannot be sampled in real time, making it difficult for online monitoring.

One of the highly effective, painless approaches to measure biomarkers levels inside the ISF is the use of microneedles (MNs). Such sharp microscopic structures are barely visible and can easily puncture the *stratum corneum* (the outermost layer of the skin) and reach the viable epidermis to enable real-time analyte measurements.

Nevertheless, most common MNs are typically constrained to rigid structures, limiting their use in stretchable or flexible devices. Indeed, the design and development of flexible microneedle devices has been challenged by low mechanical stability, breakability upon attachment to skin, making them unsuitable for long-term monitoring or for real-life clinical applications (L. Ren, et al., Fabrication of Flexible Microneedle Array Electrodes for Wearable Bio-Signal Recording. Sensors 18, 1191 (2018); K. Takeuchi et al., Flexible and porous microneedles of PDMS for continuous glucose monitoring. Biomedical Microdevices 22, 79 (2020)). Additionally, rigid MNs can easily detach from the skin and are not stable for long term measurements. Fabricating fully stretchable-soft microneedles patches typically results in mechanically weak structures that can break easily when touching the skin, and not able to reach the ISF and provide accurate measurement of the biomarkers for long periods of time.

Ciui et al., 2018 developed a wearable bendable bandage-based sensor and a minimally invasive microneedle biosensor for rapid screening of skin melanoma by detecting the presence of the tyrosinase (TYR) enzyme cancer biomarker in the presence of its catechol substrate, immobilized on the transducer surface. To overcome the mechanical challenge, the TYR bandage sensor was fabricated by screen printing stress enduring inks directly onto the soft fabric of a medical bandage. The utility of the developed microneedle platform was confirmed *in vitro* in porcine skin tissues, while a flexible ultralight electronic board was used for wireless data collection, processing, and transmission. However, although the fabrication method provided high resiliency against mechanical strains, screen printing suffers from low pattern resolution and inject printing is limited by the synthesis of conductive inks and nozzle-clogging problems (Ciui, B., et al. (2018). Wearable wireless tyrosinase bandage and microneedle sensors: toward melanoma screening. Advanced healthcare materials, 7(7), 1701264).

Wang et al. (2016) fabricated a drug delivery skin patch using an array of bendable microneedles. To tolerate the deformation associated with skin stretching at the same time with maintaining the microneedles integrity when the skin patch is applied on a joint such as elbow or knuckle, the microneedle array was designed to have a soft base and a rigid sharp tip. In cases for which the skin patch is applied on the arm or abdomen, this design is said to enable the microneedle to be dragged out of the skin instead of leaving a broken needle in it when lateral movement between the microneedle patch and skin surface occurs (Wang, H., et al. (2016). Toward self-powered wearable adhesive skin patch with bendable microneedle array for transdermal drug delivery. Advanced Science, 3(9), 1500441).

Numerous methods for making flexible microneedles were examined aiming to resolve the mechanical conflict. Typically, these methods were costly or involved multiple complex preparation steps (R. Wang et al. (2012) "A Flexible Microneedle Electrode Array With Solid Silicon Needles," in Journal of Microelectromechanical Systems, vol. 21, no. 5, pp. 1084-1089; Srivastava, A. K., et al. (2015). Long term biopotential recording by body conformable photolithography fabricated low-cost polymeric microneedle arrays. Sensors and Actuators A: Physical, 236, 164-172).

In cases where the MNs has to be used as a transistor, contact of the ISF with the device increases the potential effect of the noises on the electrical measurement. However, potentiometric sensing based on field-effect transistors (FETs) has shown promise for advanced biosensing. In comparison with other techniques, such FET platforms have the advantage of fast response time, high sensitivity, and easy fabrication with well-established electronic manufacturing processes. Up to now, FET biosensors have been developed for many analytes, most of these designs are based on flat FETs and rigid devices, yet some attempts have been made to conform the FETs to more flexible devices.

Kajisa and Sakata (2017) developed highly sensitive and biocompatible glucose sensor using a FET with a functionalized hydrogel to be applied in wearable devices to detect glucose in biological fluids. This platform was proven to be suitable also for the highly sensitive detection of biological fluids with a low glucose concentration (Kajisa, T & Sakata, T. (2017) Glucose-responsive hydrogel electrode for biocompatible glucose transistor, Science and Technology of Advanced Materials, 18:1, 26-33, DOI: 10.1080/14686996.2016.1257344).

Matsumoto et al., 2009 prepared a totally synthetic, phenylboronic acid-based glucose-sensing FET. Phenylboronic acid was chemically introduced onto the FET gate surface in the form of a thin copolymer gel layer (Current Applied Physics, 9, Supplement, 2009, Pages e214-e217).

Another study describes a device that was developed for precise, controlled and painless extraction of blood dosage and was integrated with an electrolyte-monitoring system consisting of Ion Selective Field Effect Transistor (ISFET) for measuring the concentration level of Na⁺, K⁺ and Cl⁻ in blood. The sensing elements comprised 5x5 mm² area of PMMA hollow microneedles fabricated by Synchrotron Radiation deep X-ray lithography (S. Khumpuang et al., "Development of Bio-chemical Sensor System Integrated with Blood Extraction Device," 2007 2nd IEEE International Conference on Nano/Micro Engineered and Molecular Systems, 2007, pp. 847-850).

WO2019044993 A1 discloses a device for detecting, diagnosing, and/or treating a disease in the skin or the state of skin, the device comprising: a microneedle array provided with a plurality of microneedles; labeled molecules attached to the microneedles; and a detection unit that detects, as an electrical signal, a change produced due to the labeled molecules attached to the microneedles being separated from the microneedles. US2021321942 A1 discloses a device for biomonitoring a user's health comprising a patch including a substrate configured to couple to the user's skin, and an array of microneedles carried by the substrate. The array of microneedles can be configured to access interstitial fluid in the user's skin and generate a first electrical signal indicative of at least one analyte in the interstitial fluid. Minami et al. (Tsuyoshi Minami et al, Chem. Commun., 2014,50, 15613-15615) describe an extended-gate type organic field effect transistor functionalised by phenylboronic acid for saccharide electrically detecting in water. Koklu et al, (Anil Koklu et al, Chemical Reviews Vol 122(4), 4581-4635, October 5, 2021) reviewed electrochemical detection of metabolites for early diagnosis and monitoring of a variety of health conditions. The authors focused on organic electronic material-based metabolite sensors and highlighted their potential to tackle critical challenges associated with metabolite detection.

There remains an unmet need for a FET-biosensor design with a stable MNs structure which should be stretchable and have high skin conformability, while also being rigid and strong, in order to allow MNs penetration of the outer skin layer. Such wearable device should allow accurate measurement of extremely low concentrations of various biomarkers found in the interstitial fluid via a painless test, which can *inter alia* be employed for long term health monitoring.

### SUMMARY OF THE DISCLOSURE

The present disclosure provides a wearable device for real-time detection of biomarkers from interstitial fluids. According to some examples, the biosensor device is stretchable, stable, biocompatible, and minimally invasive and allows continuous detection and monitoring of various constituents of interstitial fluids that are important for subject's health and well-being. The device is based on a new architecture of field effect transistors (FETs) which sensing part relies on an extended gate (EG) comprising microneedles (MNs) that penetrate the skin to reach the ISF to measure biomarkers found therein. In this manner, the sensing part comprising MNs could be separated from the FET transducer part, which keeps the biosensor operating in a clean, noise-free environment with high sensitivity and stability. Furthermore, integration of the microneedles obviates the need for painful extraction methods and devices and allows real time in-vivo monitoring. Comparing with a flat extended gate FET sensor, the MNs-based extended gate field effect transistor (EGFET) according to the principles of the present disclosure showed ultra-sensitivity and 10⁸ times-higher response amplitude when applied onto dry skin surface. Additional advantageous feature of the EGFET device is that the sensing part can be disposed after use and the FET can be combined with a new extended gate. The present disclosure further provides a fast, easy and inexpensive method of fabrication of the EGFET biosensor, and in particular, of its sensing part.

The sensing part (i.e., the extended gate) of the biosensor is based on microneedles which are supported on a stretchable and flexible substrate, which conforms to skin and can be thus worn comfortably for long periods of time. The inventors have surprisingly discovered that implementing a thickness-gradient strategy to combine a rigid array of microneedles and a soft stretchable substrate conveniently solved the long-standing problem of using MNs in wearable devices. In particular, the higher thickness of the substrate in a portion of the substrate on which the MNs are disposed provided the required rigidity, which protected the MNs from deformation and breaking and the lower thickness of the remaining patch afforded for the comfortable attachment to the skin and also provided electrical connection to the FET. The MNs were connected to the FET via a specially designed electric contact in a form of an elongated electrode disposed on the lower-thickness partition of the substrate, which maintained its high conductivity under bending, twisting, and stretching of the substrate. Accordingly, the microneedle-loaded stiffness-tunable patch was shown to have good stretchability, flexibility, and conductivity at the same time. The EGFET device according to the principles of the present disclosure can be used in various continuous and online health monitoring applications where conformal MNs are required, such as, but not limited to, monitoring concentrations of various body electrolytes, sugars, and hormones.

According to one aspect, there is provided a wearable extended gate field effect transistor (EGFET) device for detecting and/or measuring a constituent of an interstitial fluid from the skin of a subject, the device comprising a polymeric substrate for fixing to the skin of the subject; an extended gate electrode comprising a first microneedle configured for accessing the interstitial fluid and a first electric contact, wherein the first microneedle and the first electric contact are disposed on the polymeric substrate; and a field effect transistor (FET) associated with the first microneedle through the first electric contact.

According to some examples, the device further comprising a reference electrode comprising a second microneedle and a second electric contact, wherein the second microneedle and the second electric contact are disposed on the polymeric substrate.

According to some examples, the polymeric substrate has a thickness gradient between the first microneedle and the first electric contact.

According to some examples, the thickness of a portion of the polymeric substrate on which the first microneedle is disposed is at least 50% higher than the thickness of a portion of the polymeric substrate on which the first electric contact is disposed.

According to some examples, the portion of the polymeric substrate on which the first microneedle is disposed is substantially rigid. According to some examples, the portion of the polymeric substrate on which the second microneedle is disposed is substantially rigid.

According to some examples, the portion of the polymeric substrate on which the first electric contact is disposed is substantially flexible and stretchable. According to some examples, the portion of the polymeric substrate on which the second electric contact is disposed is substantially flexible and stretchable.

According to some examples, the portion of the polymeric substrate on which the first microneedle is disposed and the portion of the polymeric substrate on which the first electric contact is disposed are made of the same polymeric material. According to some examples, the portion of the polymeric substrate on which the second microneedle is disposed and the portion of the polymeric substrate on which the second electric contact is disposed are made of the same polymeric material.

According to some examples, the polymeric substrate is made of a polymeric material selected from the group consisting of styrene-block-isoprene-block styrene (SIS), 1-styrene-butadiene-styrene block copolymer (SBS), 2-styrene ethylene butylene styrene block copolymer (SEBS), polydimethylsiloxane (PDMS), polybutadiene rubber, polyurethane thermoplastic elastomer, low-density polyethylene (LDPH), polyisoprene, chloroprene rubber (CR) ,silicone rubber, and combinations and derivatives thereof. Each possibility represents a separate example of the disclosure. In some exemplary examples, the polymeric substrate is made of styrene-block-isoprene-block styrene (SIS).

According to some examples, the first microneedle, the second microneedle or both are made of a material selected from the group consisting of a polymer, metal, metal alloy, carbon, and combinations thereof. Each possibility represents a separate example of the disclosure.

In some examples, the metal is selected from the group consisting of Au, Ag, Pt, Ni, Ti, Cr, Cu, Pd, Al, combinations, and alloys thereof. Each possibility represents a separate example of the disclosure.

In some examples, the polymer is selected from the group consisting of polyester, polystyrene, polycarbonate, poly (methyl methacrylate), acrylate, polyvinylpyrrolidone, epoxy-based negative photoresist, and combinations thereof. Each possibility represents a separate example of the disclosure.

In some exemplary examples, the first microneedle is made of polystyrene, coated by a metal selected from Au, Pt, and Ni. Each possibility represents a separate example of the disclosure.

According to some examples, the first microneedle is modified with a biorecognition element selected from the group consisting of an enzyme, antibody, aptamer, ion-selective membrane (ISM), protonically doped polymer, DNA, ABTS (2,2'-azino-bis(3-ethylbenzothiazoline-6-sulfonic acid)), molecularly imprinted polymer (MIP), and combinations thereof. Each possibility represents a separate example of the disclosure.

In some examples, the biorecognition element is bound to the first microneedle via a linker or is held within a supporting film or matrix.

In some exemplary examples, the biomarker is a sodium ion and the biorecognition element comprises Na ionophore X and sodium tetrakis[3,5-bis(trifluoromethyl)phenyl] borate (Na-TFPB). In some related examples, the biorecognition material is immobilized on the first microneedle by polyvinyl chloride (PVC) and bis(2-ethylehexyl) sebacate (DOS).

In some exemplary examples, the biomarker is cortisol and the biorecognition element comprises monoclonal anti-cortisol. In some related examples, the biorecognition element is immobilized on the first microneedle via a (3-aminopropyl)triethoxysilane (APTES) and glutaraldehyde linker.

In some exemplary examples, the biomarker is glucose and the biorecognition element is glucose oxidase. In some related examples, the biorecognition element is immobilized on the first microneedle by chitosan. In further examples, chitosan is mixed with carbon nanotubes.

In some exemplary examples, the biomarker is a hydronium ion and the biorecognition element is polyaniline (PANI).

According to some examples, the second microneedle is made of polystyrene coated by silver.

According to some examples, the second microneedle is modified with a metal material configured to apply voltage on the surface of the second microneedle. In certain examples, the material is selected from the group consisting of AgCl/NaCl and Au. Each possibility represents a separate example of the disclosure.

According to some examples, the gate voltage of the FET is responsive to the metal material.

According to some examples, the first microneedle, the second microneedle or both have a conical shape having a height between about 250 µm and about 5 mm and a diameter at its base between about 100 µm and 2.5 mm.

According to some examples, the extended gate electrode comprises a plurality of first microneedles disposed on the polymeric substrate and arranged in an array, wherein the FET is associated with the plurality of first microneedles through the first electric contact. According to some examples, the reference electrode comprises a plurality of second microneedles disposed on the polymeric substrate and arranged in an array.

According to some examples, the first electric contact, the second electric contact or both comprise electrically conductive elongated nanostructures. In further examples, the electrically conductive elongated nanostructures are selected from the group consisting of nanotubes, nanowires, nanoribbons, nano-whiskers, nanostrips, nanorods, and combinations thereof. Each possibility represents a separate example of the disclosure. In yet further examples, the electrically conductive elongated nanostructures are made of a material selected from the group consisting of a metal, metal alloy, carbon, and combinations thereof. Each possibility represents a separate example of the disclosure. In some exemplary examples, the first electric contact and the second electric contact comprises silver nanowires (AgNWs).

According to some examples, the FET is selected from the group consisting of a metal-oxide-semiconductor field-effect transistor (MOSFET), junctionless nanowire transistor (JLNT), metal-nitride-oxide-semiconductor transistor (MNOS), junction field-effect transistor (JFET), static induction transistor (SIT); heterostructure insulated-gate field-effect transistor (HIGFET), modulation-doped field-effect transistor (MODFET); tunnel field-effect transistor (TFET), high-electron-mobility transistor (HEMT), metal-semiconductor field-effect transistor (MESFET), nanoparticle organic memory field-effect transistor (NOMFET), graphene nanoribbon field-effect transistor (GNRFET), vertical-slit field-effect transistor (VeSFET), carbon nanotube field-effect transistor (CNTFET), organic field-effect transistor (OFET), quantum field effect transistor (QFET), Schottky-barrier field-effect transistor (SB-FET), and graphene-based field effect transistor (GFET). Each possibility represents a separate example of the disclosure. In some exemplary examples, the FET is a MOSFET.

According to another aspect, there is provided a system for detecting and/or measuring a constituent of an interstitial fluid from the skin of a subject, the system comprising: the wearable EGFET device according to the aspect and various examples hereinabove; and at least one of a control unit being in electrical communication with the EGFET device, which measures an electrical signal generated by the FET in response to an interaction between the first microneedle and said constituent; and a transmitter, which receives the electrical signal generated by the FET in response to the interaction between the first microneedle and said constituent and transmits said signal to a remote server and/or to a portable electronic device.

According to some examples, the control unit is in electrical communication with each one of the FET, extended gate electrode, and reference electrode within the EGFET device.

According to some examples, the system further comprisies a display unit in electrical communication with the control unit for displaying information related to the measuring of the electrical signal, and/or means for determining concentration of the constituent in the interstitial fluid upon receipt of the electrical signal.

According to another aspect, there is provided a method for detecting and/or measuring a constituent of an interstitial fluid from the skin of a subject, the method comprising: (a) providing the system according to the aspect and various examples hereinabove; (b) fixing the wearable EGFET to the skin of a subject, thereby enabling an interaction between the first microneedle and said constituent; (c) measuring an electrical signal generated by the FET in response to the interaction between the first microneedle and said constituent; and (d) analyzing the electrical signal by at least one of the control unit, the remote server and the portable electronic device.

According to some examples, fixing the wearable EGFET to the skin of the subject comprises directly contacting the first microneedle with the skin of the subject, wherein the measuring step is performed while the wearable EGFET is fixed to the skin of the subject.

According to some examples, analyzing the electrical signal comprises comparing the electrical signal with a calibration curve and/or reference data.

According to some examples, the method further comprises displaying information related to the measuring of the electrical signal or a result of the step of analyzing (step (d)) onto a display unit being in electrical communication with the control unit, the remote server, or the portable electronic device.

According to another aspect, there is provided a method for fabricating the wearable EGFET device according to the aspect and various examples hereinabove, the method comprising: (a) providing the first microneedle, the first electric contact, and the polymeric substrate; (b) providing the FET; (c) connecting the first electric contact with the polymeric substrate; (d) connecting the first microneedle with the polymeric substrate; and (e) associating the FET with the first microneedle.

According to some examples, steps (a) and (b) can be performed in any order. According to some examples, steps (a) and (b) are performed simultaneously.

According to some examples, the method further comprises a step of forming the second microneedle and the second electric contact, and a step of connecting the second microneedle and the second electric contact with the polymeric substrate.

According to some examples, providing the first electric contact and/or and providing the second electric contact comprises spray-coating hydrophobic electrically conductive elongated nanostructures onto a Si wafer coated with a mask having a predefined opening, peeling the mask and annealing the obtained first electric contact and/or second electric contact.

According to some examples, providing the polymeric substrate comprises forming a first substantially flexible and stretchable polymeric film and combining said film with a second substantially flexible and stretchable polymeric film, which has been pre-stretched.

According to some examples, providing the polymeric substrate comprises thickening a portion of the polymeric substrate by applying a solution of the substantially flexible and stretchable polymer onto said portion.

According to some examples, the step of connecting the first electric contact with the polymeric substrate comprises spin-coating a solution of the substantially flexible and stretchable polymer onto the first electric contact to form the first substantially flexible and stretchable polymeric film prior to combining said first substantially flexible and stretchable polymeric film with the second substantially flexible and stretchable polymeric film, which has been pre-stretched.

According to some examples, the step of connecting the first microneedle with the polymeric substrate comprises fixing the first microneedle onto the thickened portion of the polymeric substrate.

Further examples and the full scope of applicability of the present disclosure will become apparent from the detailed description given hereinafter. The invention is defined in the appended set of claims.

### BRIEF DESCRIPTION OF THE DRAWINGS

Some of the examples of the disclosure are described herein with reference to the accompanying figures. The description, together with the figures, makes apparent to a person having ordinary skill in the art how some examples may be practiced. The figures are for the purpose of illustrative description and no attempt is made to show structural details of an example in more detail than is necessary for a fundamental understanding of the disclosure. For the sake of clarity, some objects depicted in the figures are not to scale.

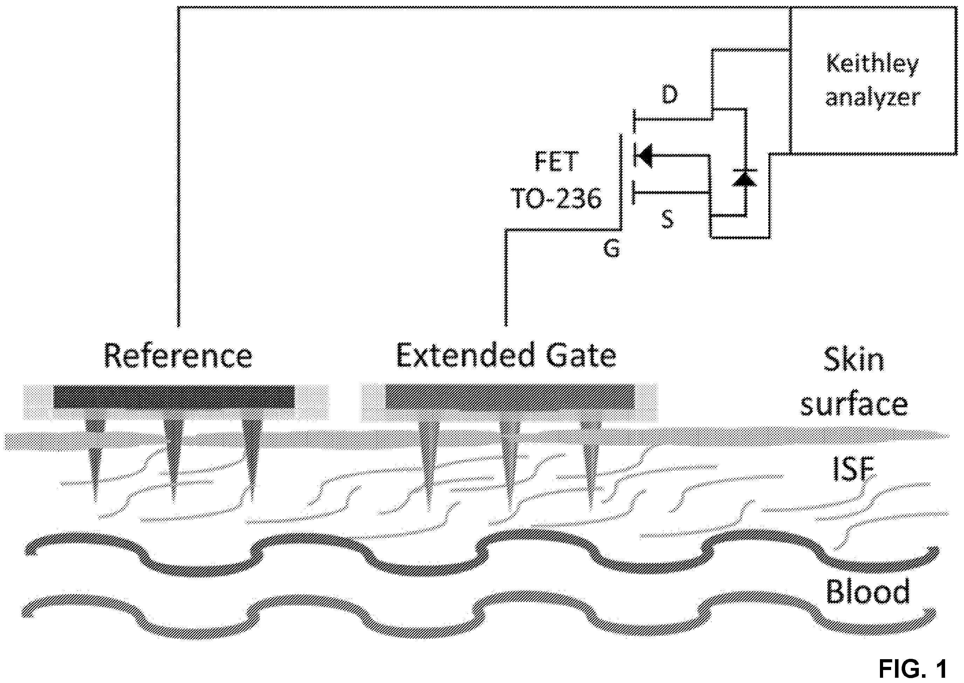

**Fig. 1****. Schematic drawing of the MN-EGFET platform and the measurement system**

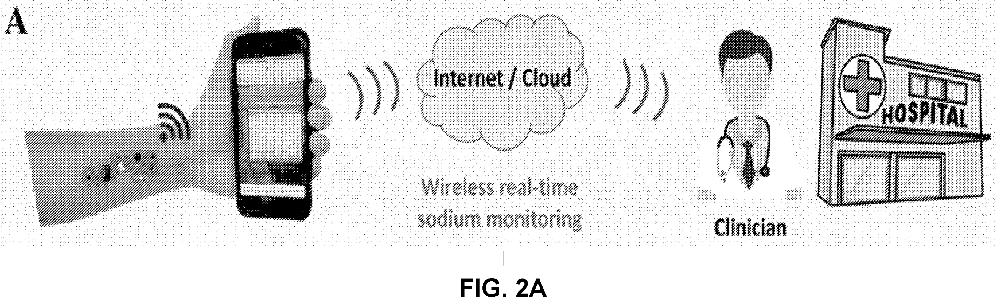

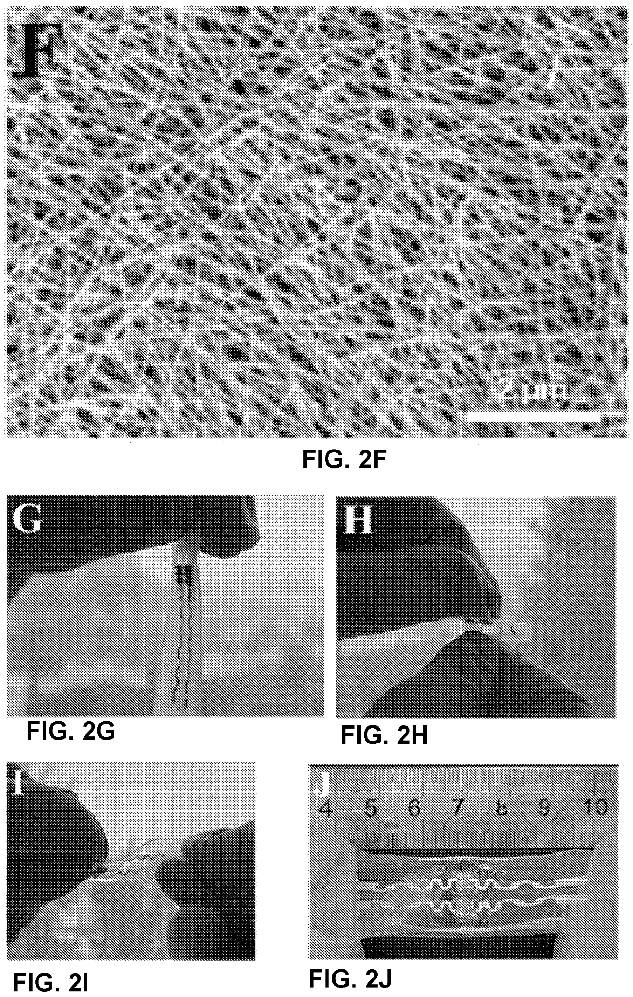

**Figs. 2A - 2J****. Design and fabrication of the MN-EGFET biosensing platform. (A)** Concept of real-time wireless monitoring of sodium levels using smartphone integrated with IoT technologies. **(B)** Illustration of the stretchable extended gate MNs and reference MNs. (C) Schematic of overall fabrication process of the MNs, starting with molding polystyrene in PDMS template, followed by Au deposition and ending with the immobilization of the antibody/enzyme/ion-selective membrane (ISM). **(D)** SEM image of the fabricated MNs. **(E)** Illustration of fabricating AgNWs electrodes on a stretchable Styrene-block-Isoprene-block Styrene (SIS) substance by using mask and spray coating strategies. **(F)** SEM image of sprayed AgNWs electrodes. **(G-I)** Photos of stretchable MN-loaded patch showing good flexibility with bending and twisting. **(J)** Stretching of the rigid MN-loaded thickness-gradient SIS film.

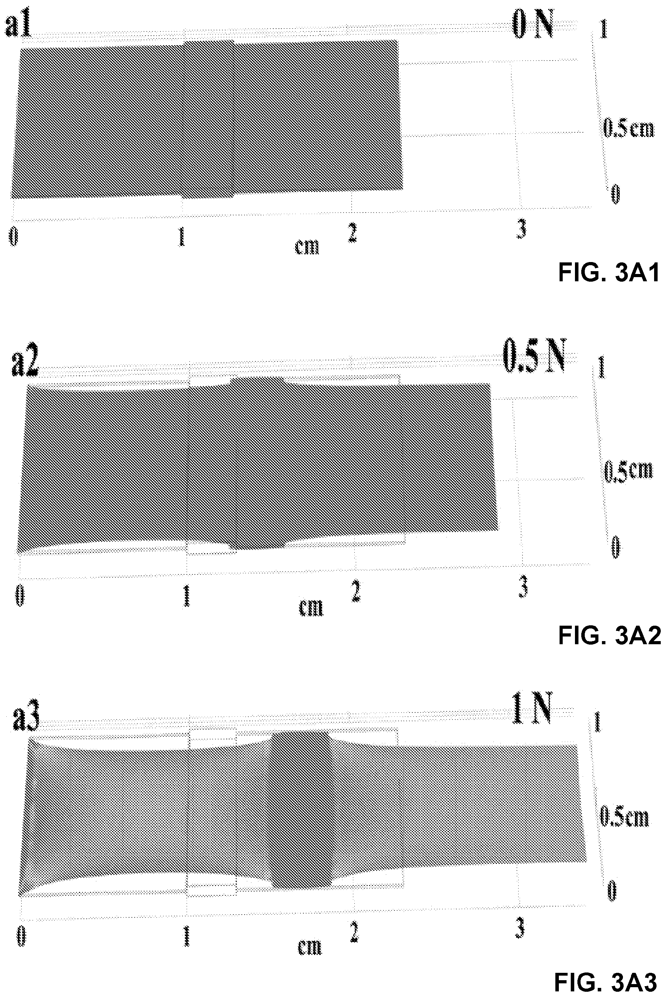

**Figs. 3A - 3B****. Thickness-gradient strategy for combining rigid MNs and stretchable patch. (A)** Tensile test simulation to investigate the stretchability of SIS polymer with different thicknesses. (B) Stretching experiments with the rigid MN-loaded thickness-gradient SIS film.

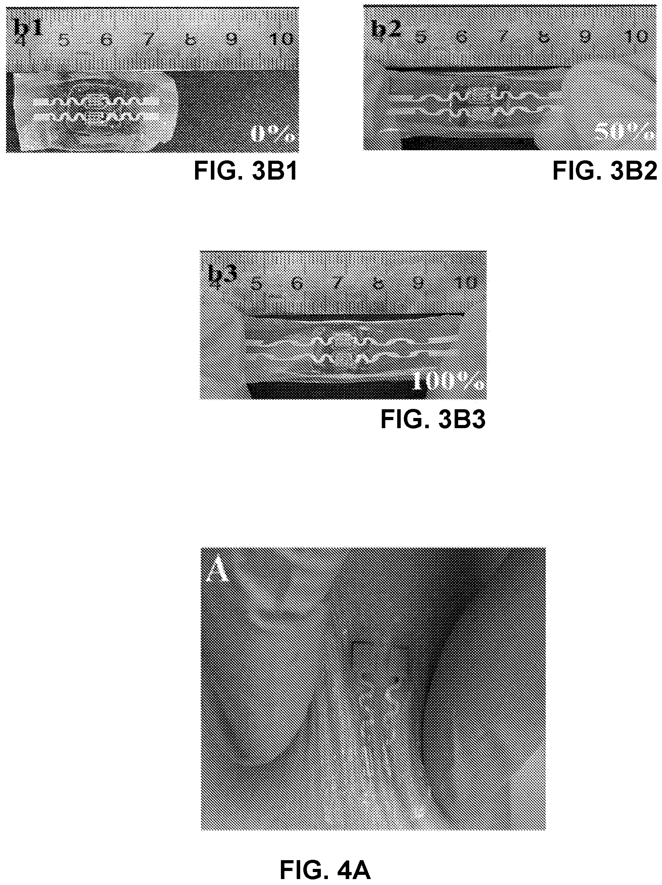

**Fig. 4A - 4I****. Performance of Sodium MN-EGFET Biosensor. (A)** Na⁺ MN-EGFET biosensor patch showing high skin conformability. **(B)** Transfer curves of the sensor. **(C)** Drain-source current curve of the sensor in response to elevated sodium concentrations. **(D)** Logarithmic correlation curve of the sensor. **(E)** Repeatability test of the sensor within 3 cycles. **(F),** Mechanical stability and sensing reproducibility of the sensor. **(G)** Selectivity test for the sensor with different analytes. **(H)** Na⁺ sensing stability under different pHs. **(I)** Drain-source current curves of the different metal EG-electrodes, Ni (black), Au (red) and Pt (Blue). Sweep cycle refers to the number of voltage sweeps from Vgs = 0 V to Vgs = 2 V with 0.05 V step.

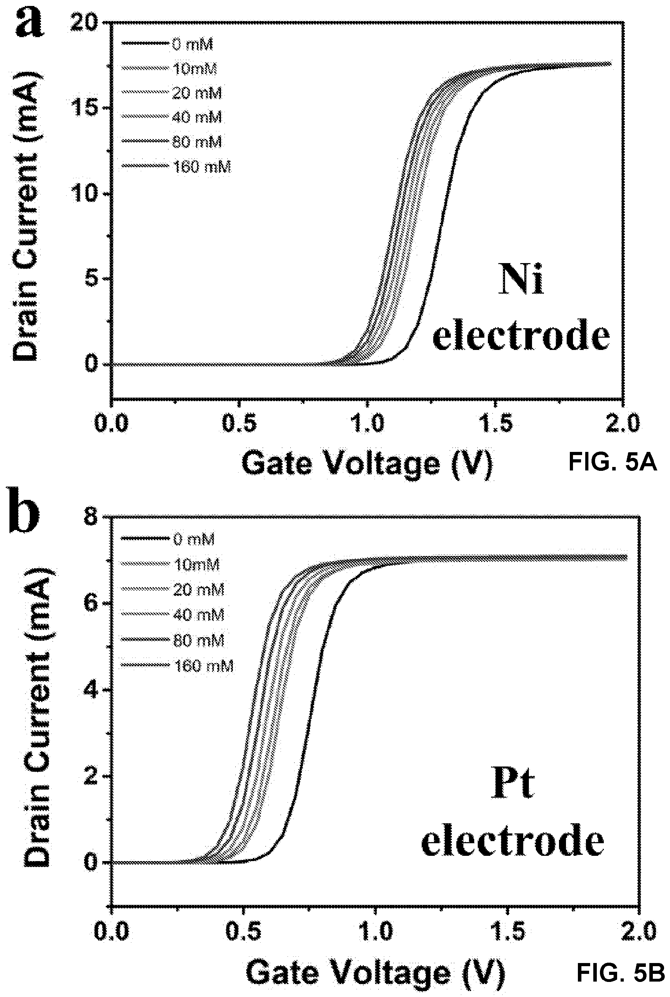

**Figs. 5A - 5D****. Sensing performance of different metal electrode-based MN-EGFET Na⁺ sensors. (A)** Transfer curves of Ni electrode **(B)** and Pt electrode based MN-EGFET Na⁺ sensors, respectively. **(C)** Threshold voltage response (ΔVth/Vth₀) curves **(D)** and drain-source current response (ΔI/I₀) curves of the different metal electrode-based MN-EGFET Na⁺ sensors.

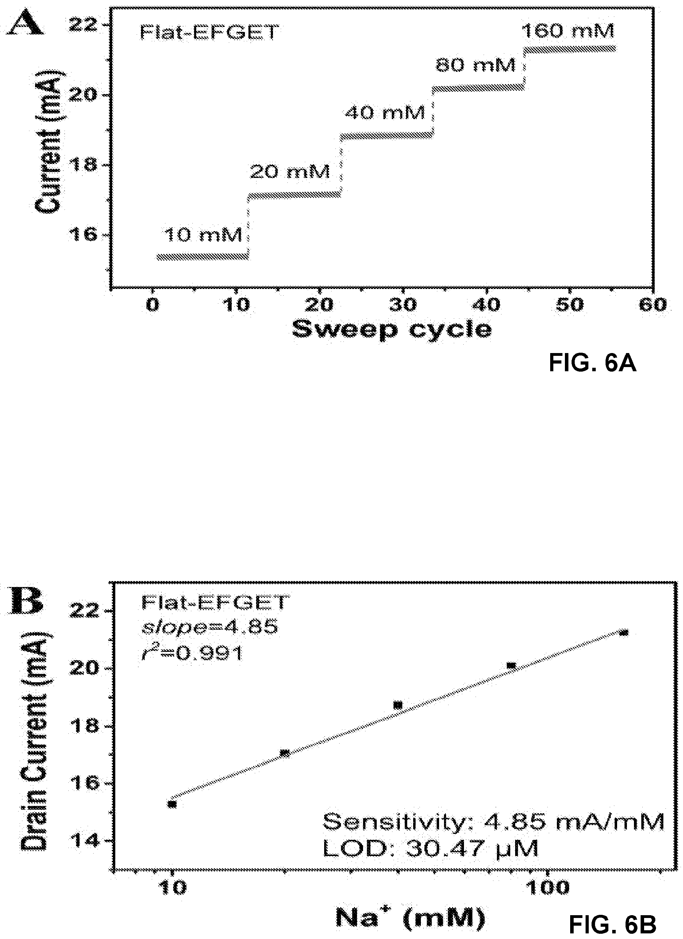

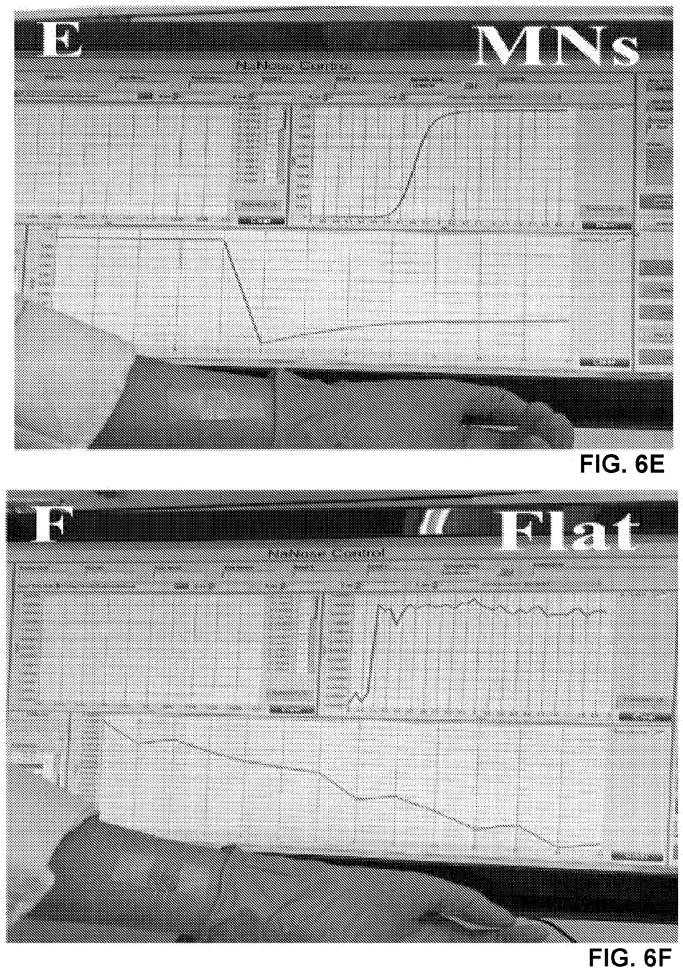

**Figs. 6A - 6G****. Comparison between flat and MN-EGFET biosensors. (A)** Drain-source current curve of the flat sensor in response to elevated sodium concentrations. **(B)** Logarithmic correlation curve of the flat sensor. Electric potential simulation of the MNs **(C)** and flat **(D)** biosensors. Real-time on-body experiment for MNs **(E)** and flat **(F)** biosensor patches. (G) Normalized response of MNs and flat patches.

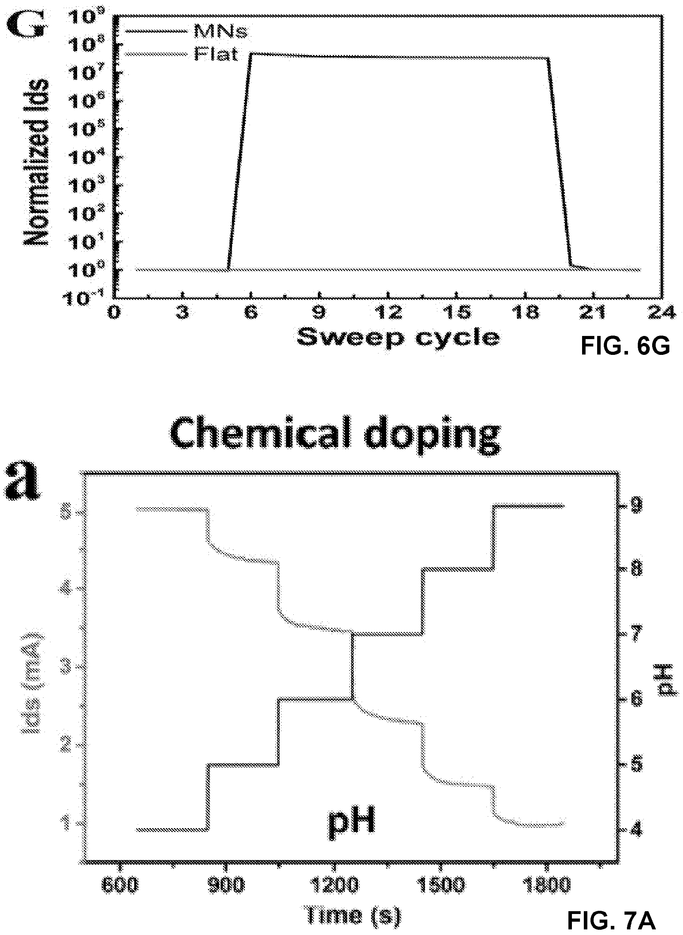

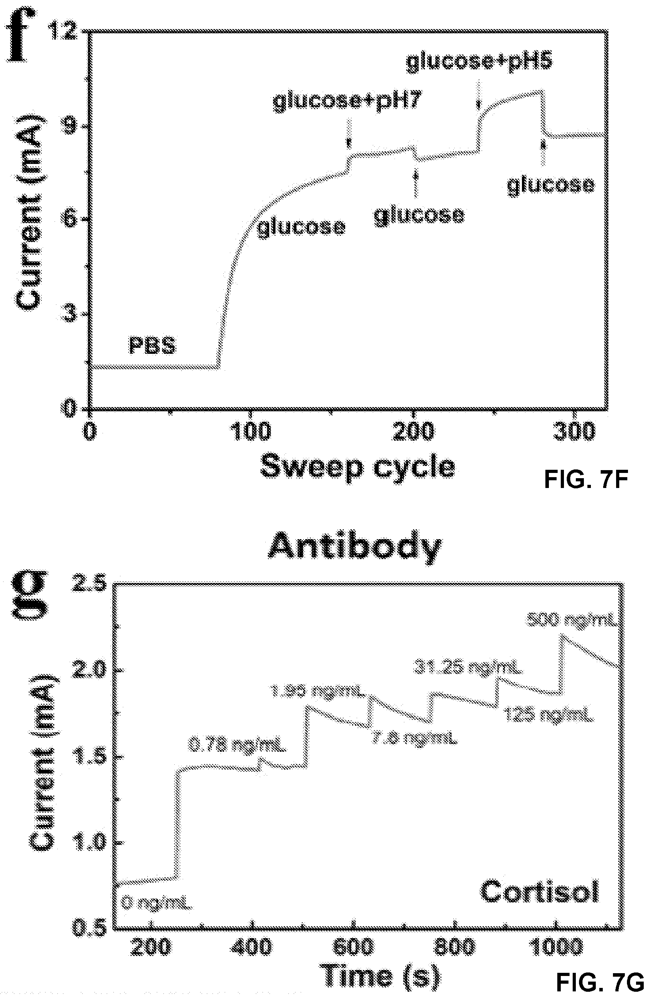

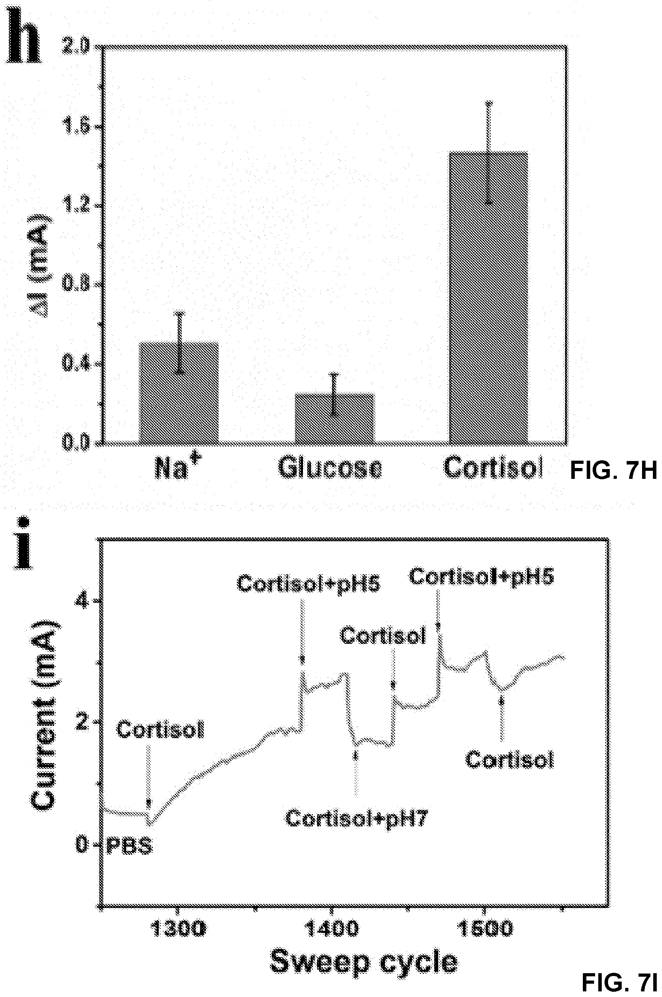

**Figs. 7A - 7I****. Toolbox for the development of various biosensors. (A)** Drain-source current responses of the pH sensor. **(B)** Selectivity performance of the pH sensor. **(C)** pH sensing stability with other biomolecules. **(D)** Drain-source current responses of the glucose sensor. **(E)** Selectivity performance of the glucose sensor. **(F)** Glucose sensing stability under different pHs. **(G)** Drain-source current responses of the cortisol sensor. **(H)** Selectivity performance of the cortisol sensor. **(I)** Cortisol sensing stability under different pHs.

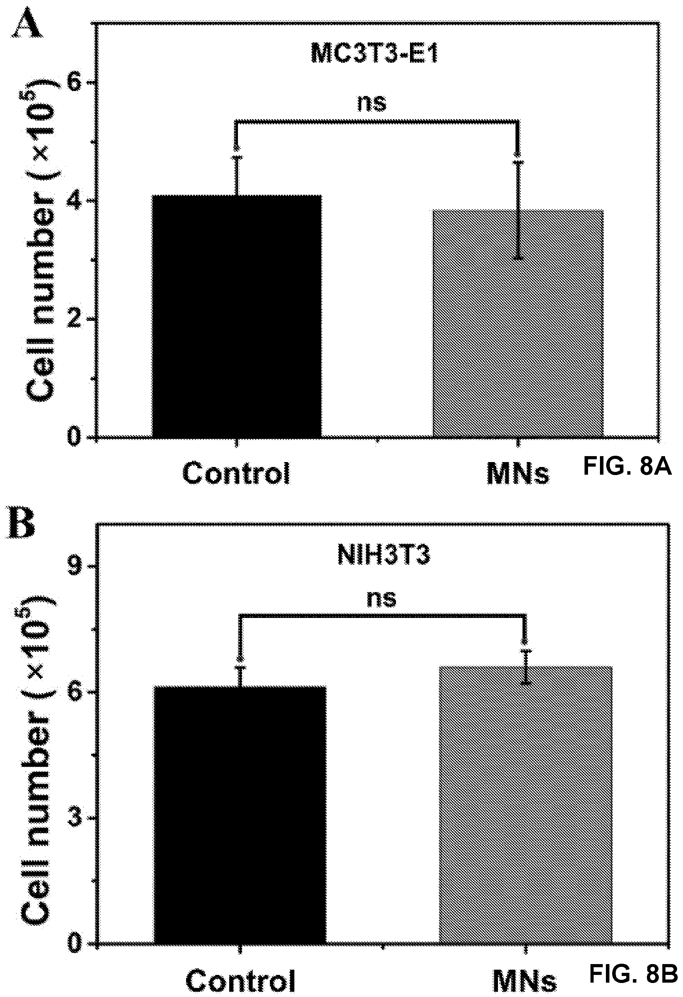

**Figs. 8A** - **8E****. *In vitro* and *in vivo* biocompatibility of MNs patch.** Cell number of MC3T3-E1 cells **(A)** and NIH3T3 cells **(B)** after 24 hours of culturing with/without the MNs patch. Live/Dead staining of MC3T3-E1 **(C)** and NIH3T3 cells **(D)** after 24 hours of culturing with/without MNs patch. n=3 biological replicates, ns represents no significant difference. **(E)** H&E images of the major organs (Heart, Liver, Spleen, Lung and Kidney) of mice with different insertion times of the MNs patch.

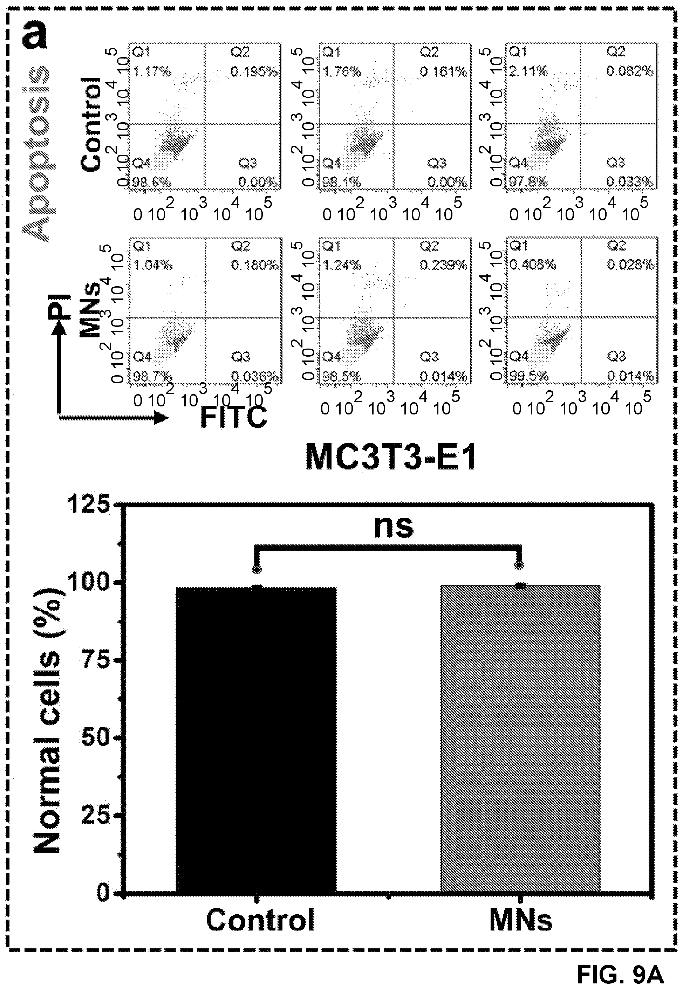

**Figs. 9A - 9D****. *In vitro* biocompatibility evaluation of the MNs patch by apoptosis assays and ROS assays. (A)** Apoptotic cells of MC3T3-E1 and **(B)** NIH3T3 cells were determined by flow cytometry after 24 hours of culturing with/without MNs patch. **(C)** ROS assays of MC3T3-E1 and **(D)** NIH3T3 cells were determined by flow cytometry after 24 hours of culturing with/without MNs patch. n=3 biological replicates, ns represents no significant difference.

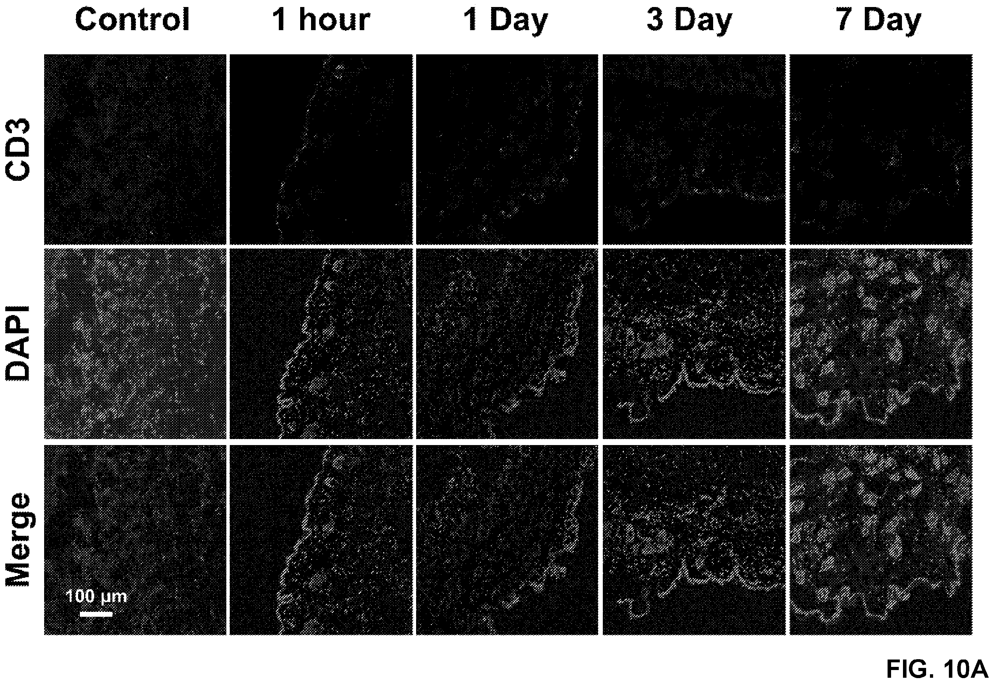

**Figs. 10A - 10B****. *In vivo* biocompatibility evaluation of the MNs patch by CD3 immunofluorescent staining.** The CD3 immunofluorescent staining of the skin tissue from each group after different insertion time. n=7 biological replicates, ns represents no significant difference.

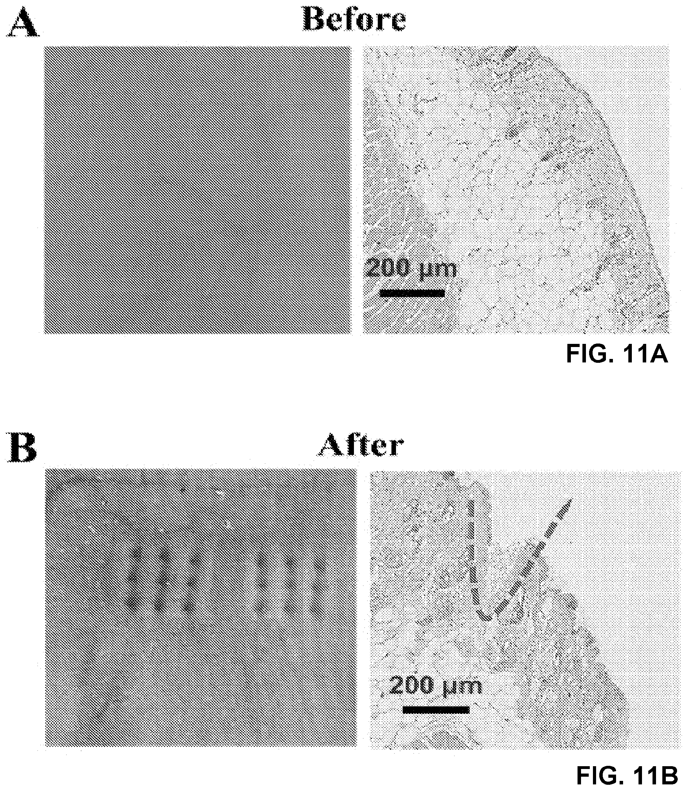

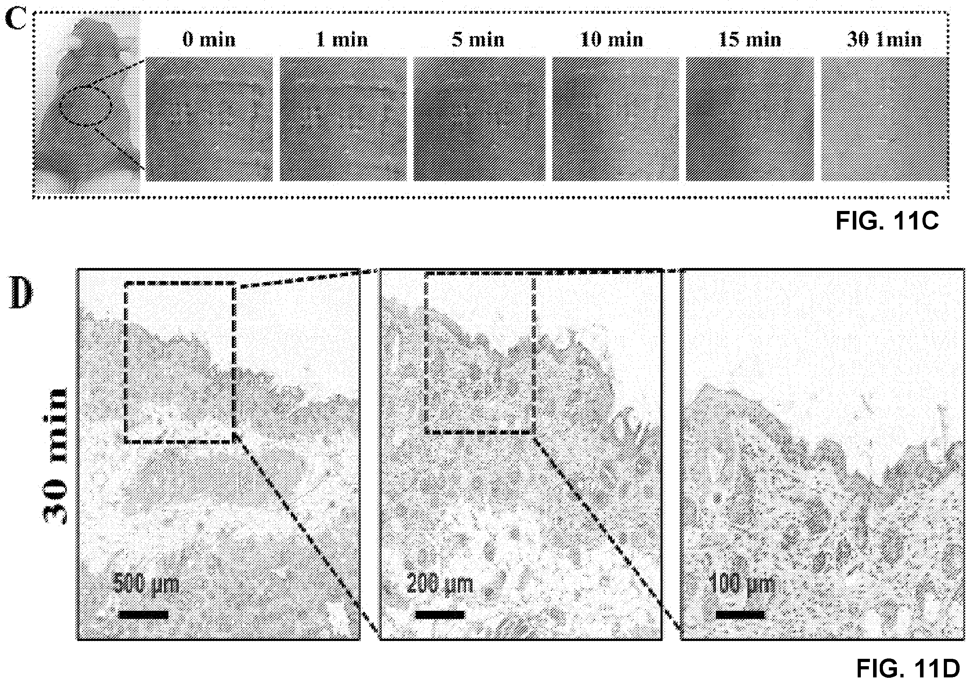

**Figs. 11A - 11D****. Skin evaluation of the MNs on mice.** Photos and H&E images of the skin tissue of mice **(A)** before and **(B)** after insertion of the MNs on the skin. **(C)** Photo of the mice after MNs insertion, including the recovery time after peeling-off the MNs, and the gradual disappearance of micro-holes over 30 min. **(D)** H&E images of the skin tissue of mice 30 min after MNs removal.

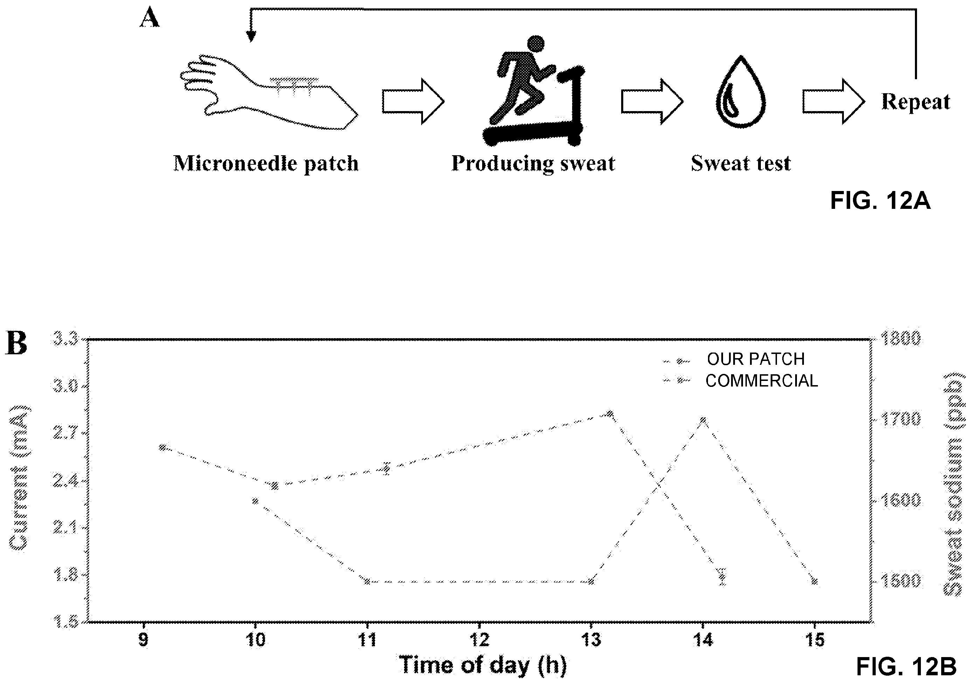

**Figs. 12A - 12F****. On-body wearable MN-EGFET patch validation. (A)** Schematic procedures of on-body study of Na⁺ sensing using the wearable MN-EGFET patch. **(B)** Long-term monitoring of Na⁺ concentration in ISF. The blue dashed line is the readout of MNs patch from ISF, and the red dashed line is the readout of a commercial device from sweat. **(C)** Concept of the wearable MN-EGFET patch for wireless health monitoring in the field of home healthcare and clinical diagnosis. **(D)** Wireless Na⁺ detection of 10 mM sodium concentration. **(E)** Wireless Na⁺ detection in the order of 10mM-40mM-160mM-10mM concentrations. **(F)** Detection data wirelessly transmitted to the smartphone.

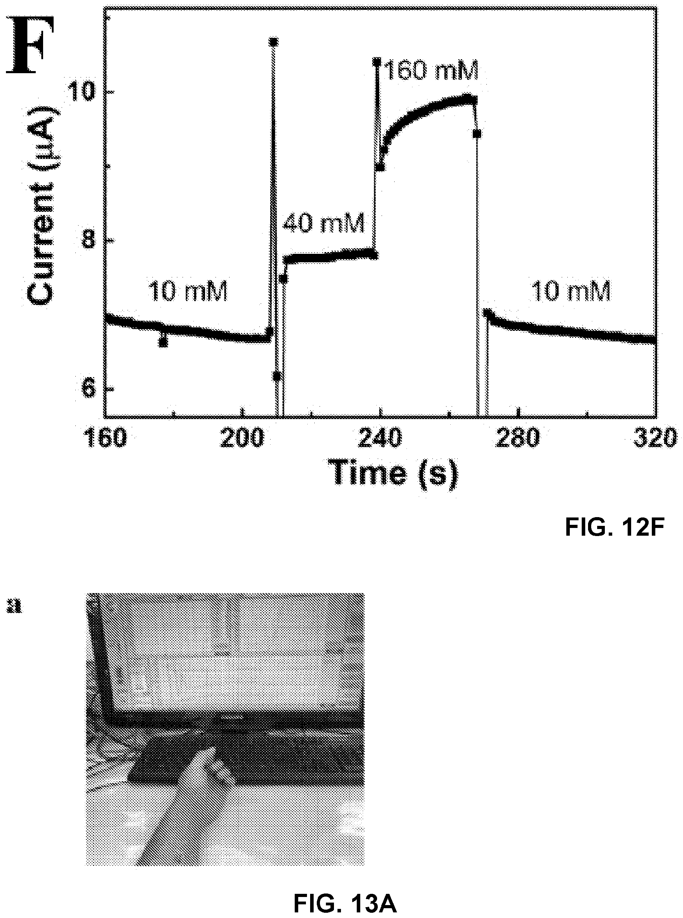

**Figs. 13A - 13F****. Details of the long-term monitoring of Na⁺ concentration in the ISF. (A)** Photo of the on-body MNs patch validation. **(B-F)** Readout of the MNs patch from the ISF at time points of 9:10(**B**), 10:10 **(C),** 11:10 **(D),** 13:10 **(E),** and 14:10 **(F).**

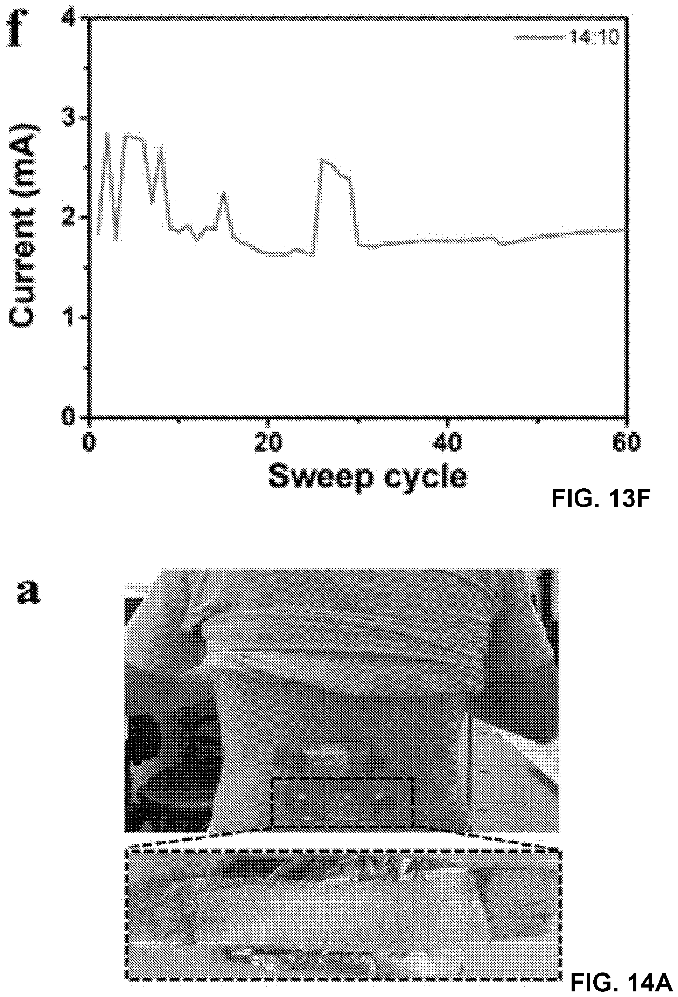

**Figs. 14A - 14G****. Details of measuring Na⁺ concentration in sweat. (A),** Photo of sweat collection from the subject's back. **(B)** Sweat measuring by commercial device (Na⁺ meter, HORIBA, B-722). **(C-G),** Readout of the commercial device from sweat at time points of 10:00 **(C),** 11:00 **(D),** 13:00 **(E),** 14:00 **(F),** and 15:00 **(G).**

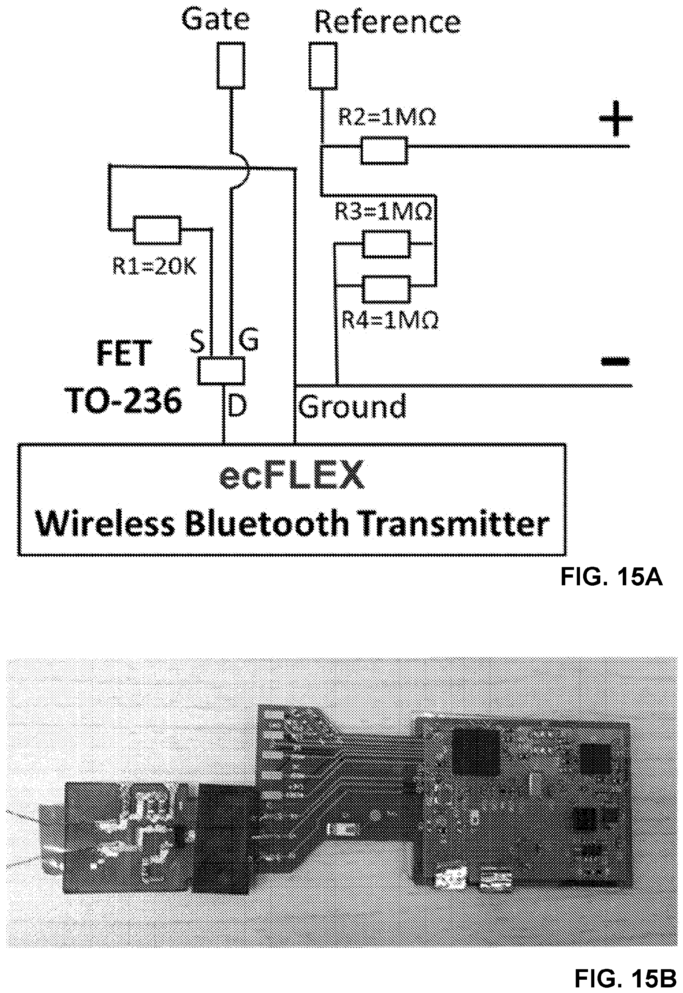

**Figs. 15A - 15B****. Circuit diagram of the connection between the MNs sensing part and the wireless Bluetooth transmitter.**

### DETAILED DESCRIPTION OF THE DISCLOSURE

In one aspect, there is provided a wearable extended gate field effect transistor (EGFET) device for detecting and/or measuring a constituent of an interstitial fluid from the skin of a subject, the device comprising: a substrate for fixing to the skin of the subject; an extended gate electrode comprising a first microneedle configured for accessing the interstitial fluid and a first electric contact, wherein the first microneedle and the first electric contact are disposed on the polymeric substrate; and a field effect transistor (FET) associated with the first microneedle through the first electric contact.

The terms "wearable" and "skin-mountable", which are used herein interchangeably, refer to a device which is configured to be placed or fixed upon the skin.

The term "interstitial fluid", as used herein, refers to the fluid that fills the spaces between cells, the fluid being composed of water, amino acids, sugars, fatty acids, coenzymes, hormones, neurotransmitters, salts, and cellular products.

The terms "constituent of an interstitial fluid" and "biomarker", which are used herein interchangeably, refer to any constituent of the interstitial fluid, which can be detected via changes of surface potential of a measuring device and/or which monitoring is of interest for evaluating health or wellbeing of a subject. Non-limiting examples of biomarkers, which can be detected by the EGFET device according to the principles of the present disclosure include glucose, cortisol, lactate, alcohol, pH, enzymes, sodium (Na⁺) and other electrolytes such as K⁺, Ca²⁺ and Mg²⁺.

The term "associated", as used herein, refers in some examples, to an electrical connection between two or more elements. In further examples, said term refers to an electrical connection, while said two or more elements are spatially separated. In some currently preferred examples, the transducer is not disposed on the polymeric substrate.

According to some examples, the device further comprises a reference electrode comprising a second microneedle and a second electric contact, wherein the second microneedle and the second electric contact are disposed on the substrate.

According to some examples, the portion of the substrate on which the first electric contact is disposed is stretchable.

The polymeric substrate comprising the first microneedle, the second microneedle, the first electric contact and the second electric contact are also termed herein "microneedle (MN) patch".

According to some examples, the substrate is a polymeric substrate. The term "polymeric substrate", as used herein, refers to a solid or semi-solid support having a surface formed of a polymer or a solid support which is formed entirely of a polymer. The term "polymer" as used herein refers generally to a macromolecule composed of repeating monomer units, and is meant to encompass natural, synthetic, and semisynthetic polymers. The term "polymeric substrate" is also meant to encompass composite polymeric materials, such as, but not limited to, polymeric materials having fillers, plasticizers, and/or fibers therein.

In some examples, the polymeric substrate is formed entirely of a polymer.

According to some examples, the polymeric substrate has a thickness gradient between the first microneedle and the first electric contact.

According to some examples, the polymeric substrate has a thickness gradient between the second microneedle and the second electric contact.

According to some examples, the thickness of a portion of the polymeric substrate on which the first microneedle is disposed is at least about 50% higher than the thickness of a portion of the polymeric substrate on which the first electric contact is disposed. In further examples, the thickness of a portion of the polymeric substrate on which the first microneedle is disposed is at least about 100%, about 200%, about 300%, about 400%, about 500% higher than the thickness of a portion of the polymeric substrate on which the first electric contact is disposed.

According to some examples, the thickness of a portion of the polymeric substrate on which the second microneedle is disposed is at least about 50% higher than the thickness of a portion of the polymeric substrate on which the second electric contact is disposed. In further examples, the thickness of a portion of the polymeric substrate on which the second microneedle is disposed is at least about 100%, about 200%, about 300%, about 400%, about 500% higher than the thickness of a portion of the polymeric substrate on which the second electric contact is disposed. According to some examples, the portion of the polymeric substrate on which the first microneedle is disposed is substantially rigid. According to some examples, the portion of the polymeric substrate on which the second microneedle is disposed is substantially rigid.

The term "substantially rigid", as used herein, refers, in some examples, to the ability of a material to resist deformation, e.g., elastic, plastic, or otherwise, wherein said resistance is adequate for the proper functioning of the microneedles, when the EGFET device is fixed to skin.

According to some examples, the portion of the polymeric substrate on which the first electric contact is disposed is substantially flexible and stretchable. According to some examples, the portion of the polymeric substrate on which the second electric contact is disposed is substantially flexible and stretchable.

The term "substantially flexible", as used herein, refers to the ability of a material to elastically deform in response to applied pressure or strain, wherein said deformation is proportional to the amount of applied pressure or strain.

The term "substantially stretchable", as used herein, refers to the ability of a material to allow a substantial increase in its length, width, and/or height in at least one spatial direction without loss of structural integrity. A substantial increase is understood to include an increase by at least 10% and may include an increase of 50%, 100%, or more.

According to some examples, the portion of the polymeric substrate on which the first microneedle is disposed and the portion of the polymeric substrate on which the first electric contact is disposed are made of the same polymeric material. According to some examples, the portion of the polymeric substrate on which the second microneedle is disposed and the portion of the polymeric substrate on which the second electric contact is disposed are made of the same polymeric material.

According to some examples, the polymeric substrate is made of a polymeric material selected from the group consisting of styrene-block-isoprene-block styrene (SIS), 1-styrene-butadiene-styrene block copolymer (SBS), 2-styrene ethylene butylene styrene block copolymer (SEBS), polydimethylsiloxane (PDMS), polybutadiene rubber, polyurethane thermoplastic elastomer, low-density polyethylene (LDPH), polyisoprene, chloroprene rubber (CR), silicone rubber, and combinations and derivatives thereof.

The term "derivative", as used herein, refers to a compound that has been subjected to one or more chemical modifications, preferably while maintaining the majority of its functionalities and structural features. Such chemical modifications include, for example, substitution, oxidation, reduction, and the like.

According to some examples, the polymeric substrate is composed of at least two layers, comprising a layer of a pre-stretched polymer and a layer of a pristine polymer (i.e., not pre-stretched). According to some examples, the polymeric substrate is composed of at least three layers, comprising a layer of a pre-stretched polymer; a layer of a pristine polymer; and an additional layer of the pristine polymer, which is located within the portion of the polymeric substrate on which the first microneedle is disposed.

According to some examples, the first microneedle, the second microneedle or both are made of a material selected from the group consisting of a polymer, metal, metal alloy, carbon, and combinations thereof. Non-limiting examples of suitable metals include Au, Ag, Pt, Ni, Ti, Cr, Cu, Pd, Al, combinations, and alloys thereof. Non-limiting examples of suitable polymers include polyester, polystyrene, polycarbonate, poly (methyl methacrylate), acrylate, polyvinylpyrrolidone, epoxy-based negative photoresist, and combinations thereof. Non-limiting examples of suitable carbons include graphite, graphene, graphene oxide, carbon nanotubes, carbon nanofibers, carbon black, fullerenes, and combinations thereof.

According to some examples, the first microneedle, the second microneedle or both are made of an insulating material coated with an electrically conductive material.

In some examples, the first microneedle is made of an Au-coated polymer, Pt-coated polymer, or Ni-coated polymer. In some exemplary examples, the first microneedle is made of polystyrene coated by a metal selected from Au, Pt, and Ni.

In some currently preferred examples, the first microneedle is modified with a biorecognition element.

The term "biorecognition element", as used herein, refers to a compound, which is selective to the target constituent (or biomarker) in the interstitial fluid. The biorecognition element can be, for example, a receptor or a probe molecule.

According to some examples, the biorecognition element is selected from the group consisting of an enzyme, antibody, aptamer, ion-selective membrane (ISM), protonically doped polymer, DNA, ABTS (2,2'-azino-bis(3-ethylbenzothiazoline-6-sulfonic acid)), molecularly imprinted polymer (MIP), and combinations thereof.

The biorecognition element can be bound to the first microneedle via a linker. In some examples, the biorecognition element is held within a supporting film or matrix.

In some exemplary examples, the biomarker is a sodium ion and the biorecognition element comprises Na ionophore X and sodium tetrakis[3,5-bis(trifluoromethyl)phenyl] borate (Na-TFPB). In some related examples, the biorecognition material is immobilized on the first microneedle by polyvinyl chloride (PVC) and bis(2-ethylehexyl) sebacate (DOS).

In some exemplary examples, the biomarker is cortisol and the biorecognition element comprises monoclonal anti-cortisol. In some related examples, the biorecognition element is immobilized on the first microneedle via a (3-aminopropyl)triethoxysilane (APTES) and glutaraldehyde linker.

In some exemplary examples, the biomarker is glucose and the biorecognition element is glucose oxidase. In some related examples, the biorecognition element is immobilized on the first microneedle by chitosan. In further examples, chitosan is mixed with carbon nanotubes.

In some exemplary examples, the biomarker is a hydronium ion and the biorecognition element is polyaniline (PANI).

According to some examples, the second microneedle is made of polystyrene coated by silver.

According to some examples, the second microneedle is modified with a material configured to maintain a constant electrical potential on the surface of the second microneedle. According to further examples, the material is selected from the group consisting of AgCl/NaCl and Au.

According to some examples, the first microneedle has a conical shape having a height between about 250 µm and about 5 mm and a diameter at its base between about 100 µm and 2.5 mm.

According to some examples, the second microneedle has a conical shape having a height between about 250 µm and about 5 mm and a diameter at its base between about 100 µm and 2.5 mm

According to some examples, the first microneedle comprises a plurality of first microneedles disposed on the polymeric substrate. In further examples, the FET is associated with the plurality of first microneedles through the first electric contact. In certain examples, the plurality of first microneedles are arranged in an array. In some exemplary examples, the plurality of first microneedles are arranged in a 3X3 array.

According to some examples, the plurality of first microneedles are electrically connected therebetween. According to further examples, the plurality of first microneedles are disposed on an electrically conducting layer disposed on the polymeric substrate.

According to some examples, the second microneedle comprises a plurality of second microneedles disposed on the polymeric substrate. In certain examples, the plurality of second microneedles are arranged in an array. In some exemplary examples, the plurality of second microneedles are arranged in a 3X3 array.

According to some examples, the plurality of second microneedles are electrically connected therebetween. According to further examples, the plurality of second microneedles are disposed on an electrically conducting layer disposed on the polymeric substrate.

According to some examples, the first electric contact, the second electric contact or both comprise electrically conductive elongated nanostructures. According to further examples, the electrically conductive elongated nanostructures are selected from the group consisting of nanotubes, nanowires, nanoribbons, nano-whiskers, nanostrips, nanorods, and combinations thereof. In yet further examples, the elongated nanostructures are made of a material selected from the group consisting of a metal, metal alloy, carbon, and combinations thereof.

In some exemplary examples, the first electric contact and the second electric contact comprises silver nanowires (AgNWs).

According to some examples, the first electric contact is elongated, being at least about 10 times longer than the diameter of the first microneedle at its base.

According to some examples, the second electric contact is elongated, being at least about 10 times longer than the diameter of the second microneedle at its base.

As explained hereinabove, the extended gate electrode is configured to sense the target biomarker. When the biomarker interacts with the first microneedle, wherein said interaction involves electron transfer, or wherein said biomarker is charged, said interaction changes the surface potential of the extended gate, which, while not being physically connected with a transducer, is electrically connected therewith. In some currently preferred examples, the transducer is a FET. In certain such examples, the change in the charge distribution of the extended gate can change the charge distribution of the semiconductor of the FET, resulting in a change in conductance of the FET channel, thereby providing detection of the biomarker, wherein the extent of the change in conductance can be used to assess the concentration of the biomarker. According to some examples, the FET is selected from the group consisting of a metal-oxide-semiconductor field-effect transistor (MOSFET), junctionless nanowire transistor (JLNT), metal-nitride-oxide-semiconductor transistor (MNOS), junction field-effect transistor (JFET), static induction transistor (SIT); heterostructure insulated-gate field-effect transistor (HIGFET), modulation-doped field-effect transistor (MODFET); tunnel field-effect transistor (TFET), high-electron-mobility transistor (HEMT), metal-semiconductor field-effect transistor (MESFET), nanoparticle organic memory field-effect transistor (NOMFET), graphene nanoribbon field-effect transistor (GNRFET), vertical-slit field-effect transistor (VeSFET), carbon nanotube field-effect transistor (CNTFET), organic field-effect transistor (OFET), quantum field effect transistor (QFET), Schottky-barrier field-effect transistor (SB-FET), and graphene-based field effect transistor (GFET).

According to some exemplary examples, the FET is a MOSFET.

According to some examples, the extended gate electrode and/or the reference electrode are disposable and the FET is reusable.

According to another aspect, there is provided a system for detecting and/or measuring a constituent of an interstitial fluid from the skin of a subject, the system comprising: the wearable EGFET device according to the aspect and various examples hereinabove; and at least one of a control unit being in electrical communication with the EGFET device, which measures an electrical signal generated by the FET in response to an interaction between the first microneedle and said constituent; and a transmitter, which receives the electrical signal generated by the FET in response to the interaction between the first microneedle and said constituent and transmits said signal to a remote server and/or to a portable electronic device.

The term "interaction", as used herein, refers to either a physical contact, such as, e.g., binding, or a chemical reaction taking place between the first microneedle and the biomarker, such as, e.g., a redox reaction.

According to some examples, the control unit is in electrical communication with each one of the FET, extended gate electrode, and reference electrode within the EGFET device.

According to some examples, the system further comprises a display unit in electrical communication with the control unit for displaying information related to the measuring of the electrical signal.

According to some examples, the system further comprises means for determining concentration of the constituent in the interstitial fluid upon receipt of the electrical signal. Said means can be a processing unit or a computer.

In another aspect, there is provided a method for detecting and/or measuring a constituent of an interstitial fluid from the skin of a subject, the method comprising: (a) providing the system according to the aspect and various examples hereinabove; (b) fixing the wearable EGFET to the skin of a subject, thereby enabling an interaction between the first microneedle and said constituent; (c) measuring an electrical signal generated by the FET in response to the interaction between the first microneedle and said constituent; and (d) analyzing the electrical signal by at least one of the control unit, the remote server and the portable electronic device.

According to some examples fixing the wearable EGFET to the skin of the subject comprises directly contacting the first microneedle with the skin of the subject.

According to some examples, analyzing the electrical signal comprises comparing the electrical signal with a calibration curve and/or reference data.

According to some examples, the method further comprises displaying information related to the measuring of the electrical signal or a result of the step of analyzing (step (d)) onto a display unit in electrical communication with the control unit, the remote server, or the portable electronic device.

According to some examples, the measuring step is performed while the wearable EGFET is fixed to the skin of the subject, the method thereby providing real-time detection and/or measurement of the constituent of the interstitial fluid.

In some exemplary examples, the biomarker is a sodium ion and the biorecognition element comprises Na ionophore X and sodium tetrakis[3,5-bis(trifluoromethyl)phenyl] borate (Na-TFPB). In some related examples, the biorecognition material is immobilized on the first microneedle by polyvinyl chloride (PVC) and bis(2-ethylehexyl) sebacate (DOS).

In some exemplary examples, the biomarker is cortisol and the biorecognition element comprises monoclonal anti-cortisol. In some related examples, the biorecognition element is immobilized on the first microneedle via a (3-aminopropyl)triethoxysilane (APTES) and glutaraldehyde linker.

In some exemplary examples, the biomarker is glucose and the biorecognition element is glucose oxidase. In some related examples, the biorecognition element is immobilized on the first microneedle by chitosan. In further examples, chitosan is mixed with carbon nanotubes.

In some exemplary examples, the biomarker is a hydronium ion and the biorecognition element is polyaniline (PANI).

In another aspect, there is provided a method for fabricating the wearable EGFET device according to the aspect and various examples hereinabove, the method comprising: (a) providing the first microneedle, the first electric contact, and the substrate; (b) providing the FET; (c) connecting the first electric contact with the substrate; (d) connecting the first microneedle with the substrate; and (e) associating the FET with the first microneedle.

According to some examples, step (a) comprises forming the first microneedle; forming the first electric contact; and forming the substrate. According to some examples, said steps can be performed in any order. According to some examples, at least two of said steps are performed simultaneously.

According to some examples, steps (a) and (b) can be performed in any order. According to some examples, steps (a) and (b) are performed simultaneously.

According to some examples, the method further comprises a step of forming the second microneedle. In some related examples, the method further comprises a step of forming the second electric contact. In further related examples, the method further comprises a step of connecting the second microneedle and the second electric contact with the substrate.

According to some examples, the step of forming the first microneedle comprises forming a mold comprising conical holes and filling said holes with a biocompatible polymer. In some related examples, the step of forming the first microneedle further comprises coating the obtained first microneedle with a metal. The metal can be selected from the group consisting of Au, Ag, Pt, Ni, Ti, Cr, Cu, Pd, Al, combinations, and alloys thereof. In further examples, the step of forming the first microneedle further comprises modifying the obtained first microneedle with a biorecognition element.

According to some examples, the step of forming the second microneedle comprises forming a mold comprising conical holes and filling said holes with a biocompatible polymer. In some related examples, the step of forming the second microneedle further comprises coating the obtained second microneedle with a metal selected from the group consisting of Au, Ag, Pt, Ni, Ti, Cr, Cu, Pd, Al, combinations, and alloys thereof. In further examples, the step of forming the second microneedle further comprises modifying the obtained second microneedle with a material configured to maintain a constant potential on the surface of the second microneedle.

According to some examples, the step of forming the first electric contact and/or the step of forming the second electric contact comprise spray-coating hydrophobic electrically conductive elongated nanostructures onto a Si wafer coated with a mask having a predefined opening, peeling the mask and annealing the obtained electric contact.

According to some examples, the substrate is a polymeric substrate. According to some examples, the step of forming the polymeric substrate comprises forming a first substantially flexible and stretchable polymeric film and combining said film with a second substantially flexible and stretchable polymeric film, which has been pre-stretched.

According to some examples, the step of forming the polymeric substrate comprises thickening a portion of the obtained polymeric substrate. According to some related examples, said thickening comprises applying a solution of the substantially flexible and stretchable polymer onto said portion.

According to some examples, the step of connecting the first electric contact with the polymeric substrate comprises spin-coating a solution of the substantially flexible and stretchable polymer onto the first electric contact to form the first substantially flexible and stretchable polymeric film prior to combining said first substantially flexible and stretchable polymeric film with the second substantially flexible and stretchable polymeric film, which has been pre-stretched. According to some examples, the polymeric film is at least about 50% pre-stretched. According to further examples, the polymeric film is at least about 100% pre-stretched.

According to some examples, the step of connecting the second electric contact with the polymeric substrate comprises spin-coating a solution of the substantially flexible and stretchable polymer onto the second electric contact to form the first substantially flexible and stretchable polymeric film prior to combining said first substantially flexible and stretchable polymeric film with the second substantially flexible and stretchable polymeric film, which has been pre-stretched.

According to some examples, the step of connecting the first microneedle with the polymeric substrate comprises fixing the first microneedle onto the thickened portion of the polymeric substrate. According to some examples, the step of connecting the second microneedle with the polymeric substrate comprises fixing the second microneedle onto the thickened portion of the polymeric substrate.

According to some examples, the step of associating the FET with the first microneedle comprises electrically connecting the FET to the first electric contact.

As used herein and in the appended claims the singular forms "a", "an," and "the" include plural references unless the content clearly dictates otherwise. Thus, for example, reference to "a microneedle" includes a plurality of such microneedles as known to those skilled in the art, and so forth. It should be noted that the term "and" or the term "or" is generally employed in its sense including "and/or" unless the content clearly dictates otherwise.

As used herein, the term "about", when referring to a measurable value such as an amount, a temporal duration, and the like, is meant to encompass variations of +/-10%, more preferably +/-5%, even more preferably +/-1%, and still more preferably +/-0.1% from the specified value, as such variations are appropriate to perform the disclosed methods.

The following examples are presented in order to more fully illustrate some examples of the disclosure. They should, in no way be construed, however, as limiting the broad scope of the disclosure. One skilled in the art can readily devise many variations and modifications of the principles disclosed herein without departing from the scope of the disclosure.

### EXAMPLES

### Example 1 - Design and fabrication of the wearable stretchable MN-EGFET device

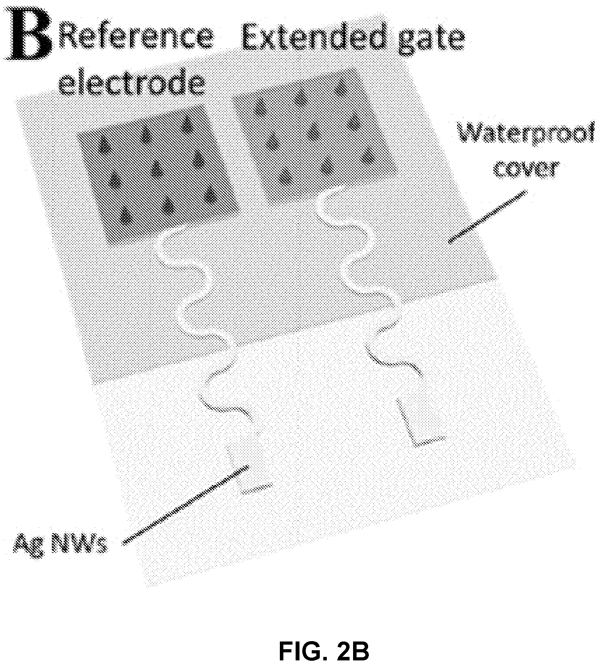

A scheme of the stretchable skin-conformal microneedle-based extended gate Field-Effect Transistor (MNs-EGFET) device and its measurement system is demonstrated in **Figure 1****.** The device is shown to comprise two separated main parts: a replaceable MNs sensing part and a reusable transducer. The sensing MNs array includes the rigid MNs EG, a reference electrode and silver nanowire (AgNW) electrodes (also termed herein "first electric contact" and "second electric contact") **(****Figure 2B****).**

In some examples, as illustrated in **Figure 1****,** the reference electrode is coupled to a voltage supply (e.g. a Keithley analyzer) to provide gate voltage to the FET. Thus, in some examples, the metal material of the microneedles of the reference electrode are configured to apply voltage on the surface of the second microneedles, and the gate voltage of the FET is responsive to the metal material of the microneedles of the reference electrode since the voltage of the reference electrode is received as input to the power supply of the FET. In some examples, the applied voltage tunes the gate in EGFET operation.

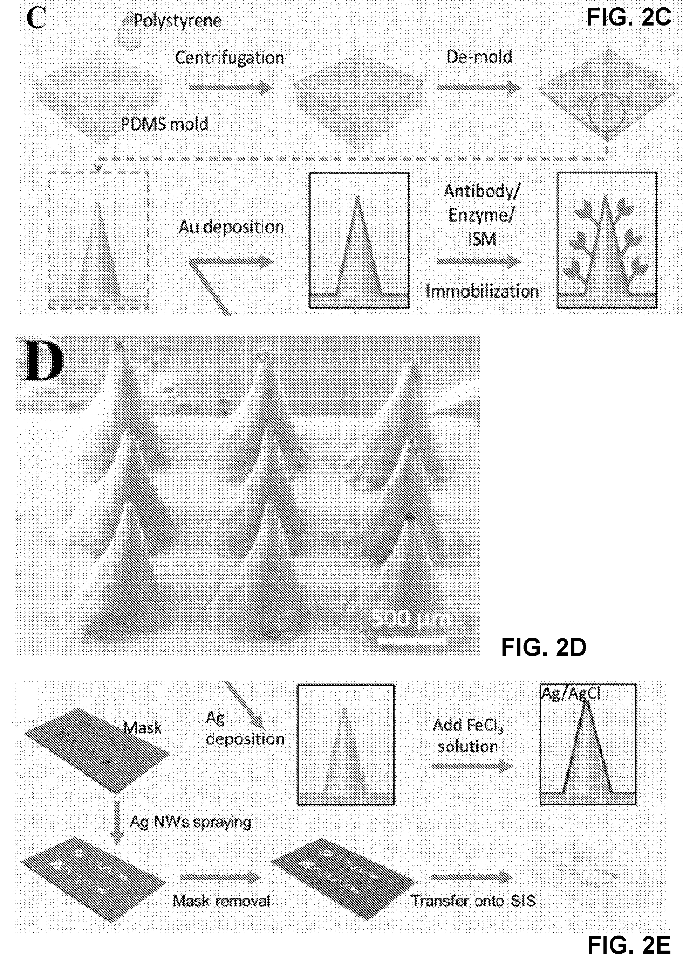

The overall fabrication process of the MNs is demonstrated in **Figure 2C****.** Molds of Polydimethylsiloxane (PDMS) for MNs fabrication were prepared by carving even holes using a laser cutter (Universal Laser Systems VersaLASER (VLS)). The PDMS mold was added to a tube filled with 200 mg/ml polystyrene in DMF solution and then were centrifuged at 3,000 rpm for 5 min to assist the solution in penetrating and filling the holes in the mold. The molds were dried at 80°C overnight to obtain a 3X3 MNs array. After demolding, the MNs were deposited with a layer of Au or Ag for EG or reference electrodes, respectively. EG electrodes were then modified by immobilizing the biorecognition element as detailed in the following examples. Ag/AgCl reference electrodes were obtained by drop-casting 10µL of 0.05M FeCl₃ solution on top of the Ag reference electrode for 1 min, which was then washed with deionized water. A solution of NaCl and polyvinyl butyral (PVB) in methanol was added before being vacuum-dried for 30 min to coat the reference electrode. SEM micrograph of the obtained MNs, taken by Zeiss Ultra Plus High-Resolution Cryo-Scanning Electron Microscope, demonstrates their conical shape with a 10 µm radius of curvature at the tip, a diameter of 500 µm at the base and ~1000 µm in height **(****Figure 2D****).**

In this design, the exceptional architecture of the MNs enables accessibility to the interstitial fluids' (ISF's) sodium and controllability of the FET's mode of operation in real-time, without the need for extraction methods or any excessive activities. Subsequently, the concentration input is transduced to an electric signal by the FET and the output can be directly transferred to doctors and clinics via smartphone/computer integrated with IoT technologies **(****Figure 2A****).**

For the synthesis of silver nanowires (AgNWs), 2.5 g of Polyvinylpyrrolidone (PVP) was added to 40mL ethyl glycol solution and mixed with 100 µL of 0.15 M FeCl₃ in ethyl glycol for 5 min at 160°C. 100 µL of 0.15M NaCl was added as a catalyst for the reaction, and then 10 mL of 1.5 M AgNO₃ was added dropwise while mixing until a color change of the mixture to light silver is preserved. Methanol was added after 2 h to stop the reaction. To obtain the AgNWs, the samples are then centrifuged to separate from the other residues. Pure AgNWs were then modified with 1H,1H,2H,2H-Perfluorodecanethiol, producing hydrophobic AgNWs-Florine (AgNWs-F). In brief, 50 mg AgNWs were suspended in 5mL ethanol/chloroform (1:1 by volume) solution and sonicated for 5 min. 5 µL of 1H,1H,2H,2H-perfluorodecanethiol was added and the mixture was sonicated for 10 min, after which it was kept stirred overnight at 90°C. The mixture was then centrifuged 3 more times with ethanol/chloroform solution (3,000 rpm for 6 min) to separate the residues and dried in a vacuum oven at 50°C.

To fabricate the rigid-soft gate structure, an electrode mask was applied on a hydrophobic Si wafer and AgNWs-F were spray-coated. After peeling off the mask, the sprayed AgNWs-F were annealed at 200 °C for 20 min to obtain a conductive AgNWs electrode. 100 mg/mL styrene-block-isoprene-block styrene (SIS) in toluene was spin-coated onto AgNWs electrode with 1,000 rpm for 60 s to fabricate stretchable thin film. This thin film was transferred onto another 100% pre-stretched SIS film prepared with Teflon template to obtain a stretchable AgNWs electrode (the soft part). To create a variant thickness substance, SIS solution was drop-casted at specific points onto the backside of a prepared stretchable AgNWs electrode to thicken an area on the film (the rigid part). The rigid MNs were then fixed on the rigid part of this thickness-gradient AgNWs electrode film using silver paint. The MNs array (the gate) integrated with the SIS substrate was then combined with the transducer device (FET TO-236) to obtain the MN-EGFET platform **(****Figure 2E****).**

### Example 2 - Physical characterization of the components of the wearable stretchable MN-EGFET device

SEM image of the sprayed AgNWs electrodes demonstrates their network structure (**Figure 2F**) that provides good conductivity, under bending, twisting, and stretching. **Figures 2G-J** show good flexibility of the MN-loaded patch with bending and twisting.

The stretchability of Styrene-block-isoprene-block styrene (SIS) polymer was investigated using a tensile test simulation as shown in **Figure 3a₁₋₃**. The middle part of the SIS film is thicker than both side parts (0.5 µm and 0.1µm, respectively). Without applying any stretching force, the polymer layer has no deformation **(Figure 3a₁**). After increasing the stretching force from 0N to 1N **(Figure 3a₂₋₃**), clear deformation could be seen in the thin layer (side part), meanwhile, there was no obvious deformation in the thick layer (middle part). Stretching experiments with rigid MN-loaded thickness-gradient SIS film as shown at **Figure 3b₁₋₃**, proved that the MNs loaded in the middle part maintained their shape, remained stable and did not detach when the patch was stretched from 0 to 100% deformation. By using the thickness-gradient strategy, the MN-loaded patch has both rigid MNs to penetrate the skin and a stretchable substrate to maintain skin conformability, demonstrating possible applications that can be used for long-term health monitoring.

### Example 3 - Preparation and performance of the Na⁺ MN-EGFET Biosensor

Na⁺ MN-EGFET biosensor was fabricated by modifying the MNs with sodium-selective membrane. For this purpose, 10 mg sodium ionophore X were mixed with 5.5 mg sodium tetrakis[3,5-bis(trifluoromethyl)phenyl] borate (Na-TFPB), 33 mg polyvinyl chloride (PVC) and 654.5 mg bis(2-ethylehexyl) sebacate (DOS) and dissolved in 6.6 mL tetrahydrofuran (THF). The solution was stored at 4°C overnight and then 5 µL was drop-casted onto the extended gate electrode.

To test its flexibility on skin surface, the Na⁺ MN-EGFET biosensor patch was placed on a dry skin and presented high skin conformability **(****Figure 4a****).**

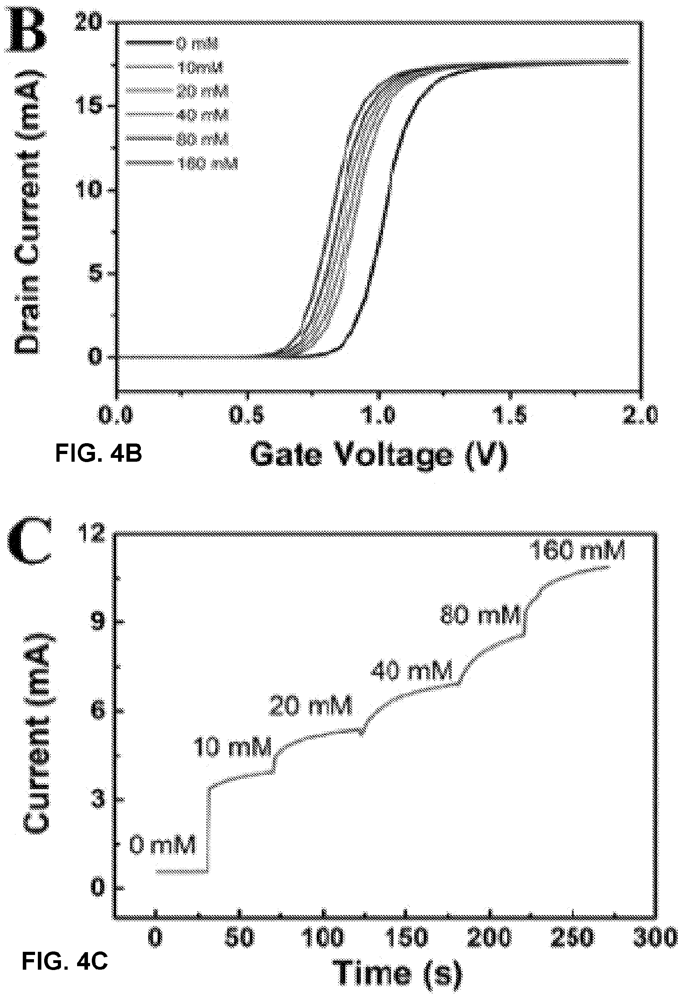

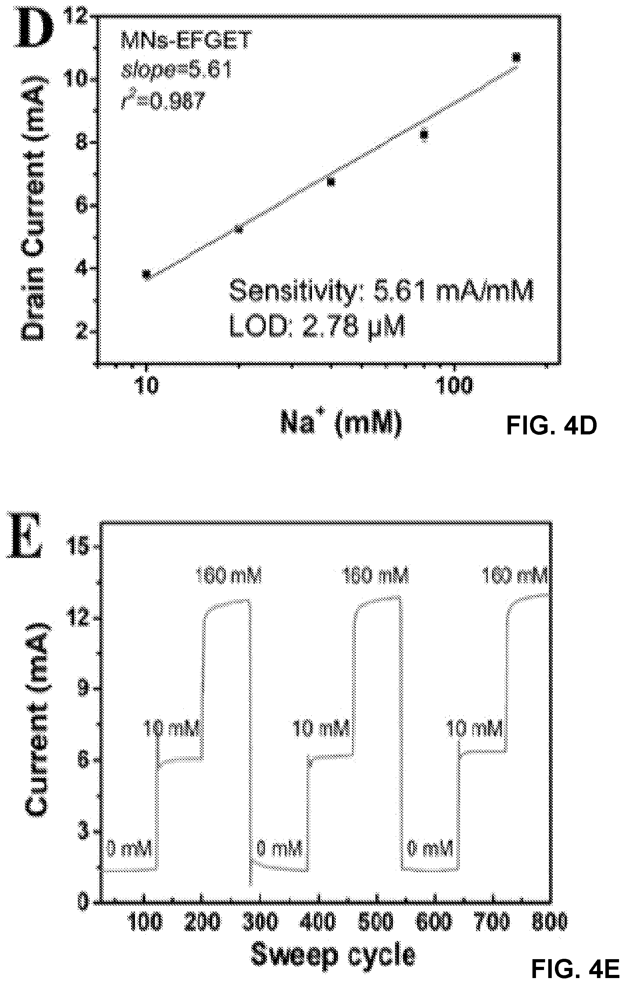

In order to evaluate the sensing performance of Na⁺ MN-EGFET, sodium solutions at a concentration range of from 10 to 160 mM were made, given that the normal average concentration of the Na⁺ in ISF is ~145 mM. The electrical behavior of the fabricated EGFET sensor was measured using Keithley 2536A System Source meter. As shown in **Figure 4B****,** the transfer curve shifted to the left with elevated sodium concentrations, indicating that the sensor was responding to changes in concentrations. Elevated response of the drain-source current was also seen with time **(****Figure 4C****),** showing a strong linear correlation (r²=0.987) in response to the elevated sodium concentrations **(****Figure 4D****).** The sensitivity of the biosensor and its limit of detection (LOD), given by the logarithmic correlation were 5.61 mA/mM and 2.78 µM, respectively.

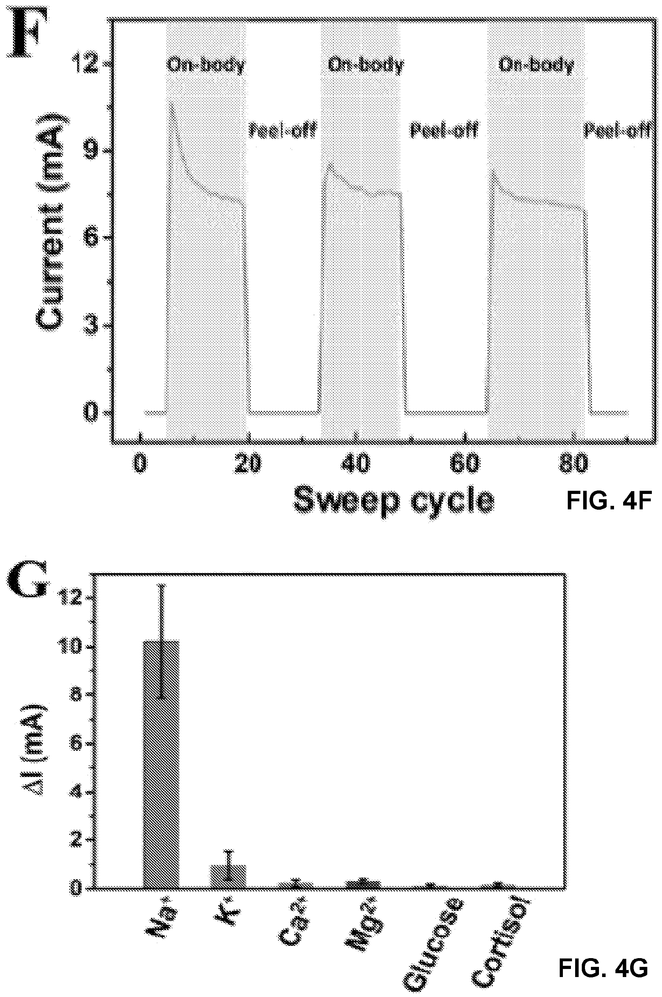

A repeatability test was conducted by applying 3 cycles of elevated sodium concentrations (10 mM and 160 mM), leading to a repeatable elevation of the drain-source current response **(****Figure 4E****),** indicating on the stability of the MN-EGFET biosensor. The biosensor has also demonstrated a mechanical stability as is shown in **Figure 4F****.** Even though the biosensor patch was placed on the body and peeled-off along 3 cycles, the response stayed stable and repeatable over sweep cycles, leading to repeatable and similar current values, thereby proving the high mechanical stability and sensing reproducibility of the MNs patch when connected and disconnected from the body.

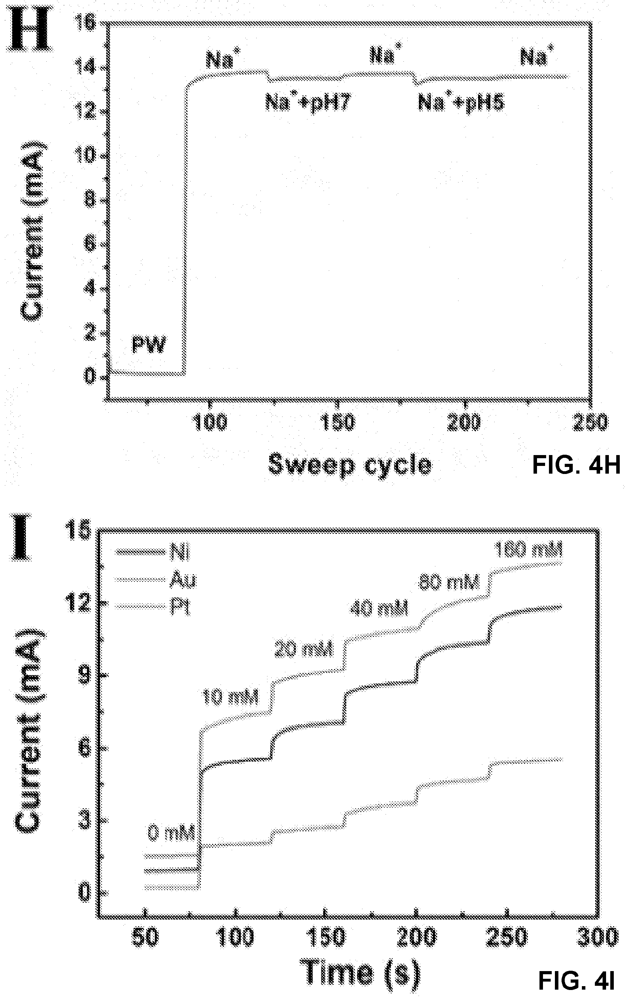

The selectivity of the Na⁺ MN-EGFET was also assessed by applying sodium and other analytes, including K⁺, Ca²⁺, Mg²⁺, glucose and cortisol. As shown in **Figure 4G****,** The Na⁺ biosensor had high selectivity for Na⁺ electrolyte, with a current change (ΔI) of 10 ± 2mA, and a low response to the other electrolytes and biomarkers (K⁺ ~ 0.9 ± 0.8mA, Ca²⁺~ 0.2 ± 0.1mA, Mg²⁺~ 0.3 ± 0.1mA, glucose ~ 0.08 ± 0.07mA and cortisol ~ 0.13 ± 0.07mA), proving the high selectivity potential of this Na⁺ MN-EGFET in measuring Na⁺ in the ISF. Furthermore, the stability of the Na⁺ biosensor at different pH values was examined, considering that normal ISF pH is 7.35-7.45, whereas in the case of health disorder or disease the pH will be lower (pH <7.35) and could become more acidic. The sensor demonstrated good stability despite the minor changes observed when changing the pH to 5 and 7 **(****Figure 4H****),** emphasizing that this sensor gives stable measurements when inserted in the ISF regardless of the pH.

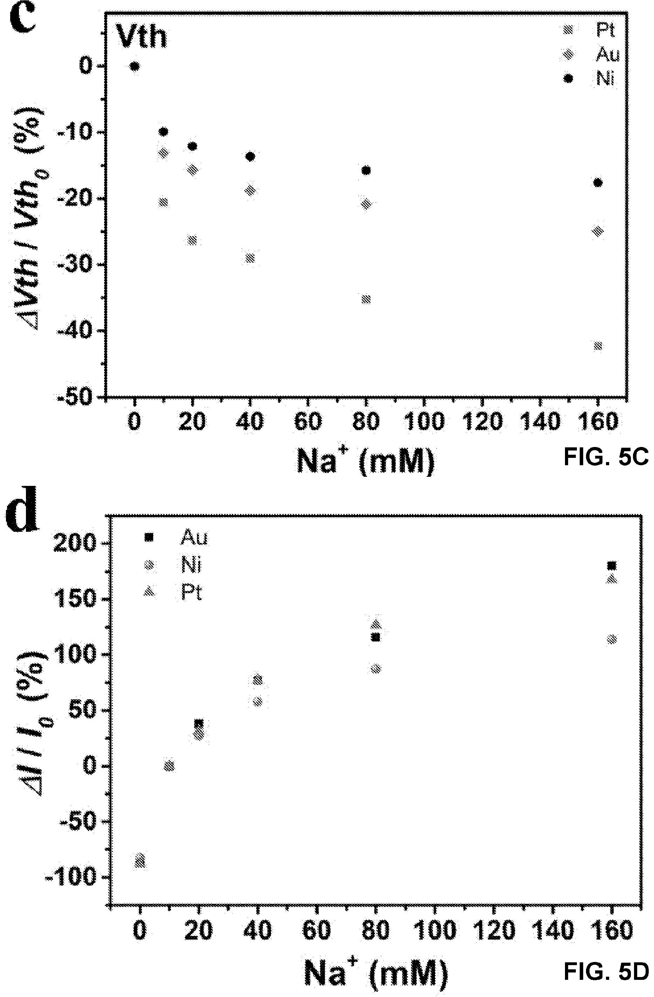

The sensing performance of different metal electrode-based MN-EGFET Na⁺ sensors by fabricating Na⁺ biosensors using different metals such as Nickel (Ni) and Platinum (Pt) was examined. As is shown in Figure 4I, Ni- and Pt- electrodes had an elevated trend and response to sodium concentration. Furthermore, Ni- and Pt- sensors were exposed to elevated concentrations of sodium analyte (from 0mM to 160mM). As shown in **Figure 5a****,** **b**, all the Ni- and Pt- electrode based sensors showed similar elevated trend and response to the sodium concentration, as well as shifted transfer curves. Ni electrode-based sensor presented higher gate voltage (Vgs) range from 0.9V to 1.3V, while Pt electrode-based sensor presented lower Vgs range from 0.3V to 0.7V. Considering Vgs range of Au electrode-based sensor is from 0.6V to 1.0V, Pt electrode-based sensor has the lowest energy consumption when working with a constant Vgs. Further analysis of the data showed that the Pt electrode sensor has the highest threshold voltage response (ΔVth/Vth₀ equal to -45% at the highest concentration of Na⁺ 160mM **(****Figure 5c****)** whereas Au's threshold voltage response is equal to -25% and Ni's is equal to -20%. The drain-source current responses (ΔI/I₀) of the different metal-electrodes were also shown in **Figure 5d****.** In this case Au electrode-based sensor showed the highest response (~175%), followed by Pt (~150%) and then Ni (~100%). These experiments demonstrate the variety of options for choosing the metal for the desired application and based on the needed electrical properties for the developed MN-EGFET biosensor.

### Example 4 - Comparison of MN-based extended gate and flat extended gate biosensor

The sensing performance of a flat EGFET sensor was measured as described in Example 1 and compared with the sensing performance of the MN-EGFET sensor. As is shown in **Figure 6A-B** the sensitivity and LOD values of the flat EGFET sensor are 4.85 mA/mM and 30.47 µM, respectively, whereas the sensitivity and LOD values of the MN-EGFET sensor are ~1.16 higher and ~11 times lower, respectively.

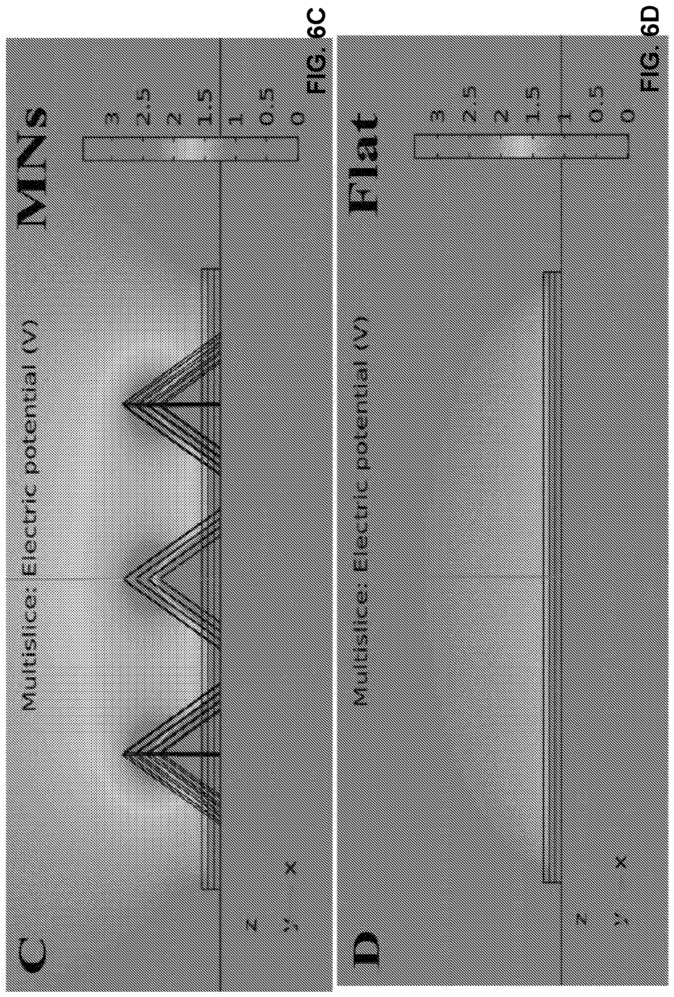

Electric potential simulation was conducted *via* COMSOL. Modifying the MNs with a functional molecular monolayer (e.g., ISM) and introducing a charged analyte (e.g., Na⁺), lead to a charged interface. The surface electric potential (V) caused by the adsorbed charged analytes, was simulated for both MNs and flat electrodes, in this case the electric potential distribution represents the positive charged sodium molecules (Na⁺). As presented in **Figure 6C****,** the electric potential for the MNs was with a wider range and a maximum value of +3V in the tip of the MNs. In accordance with previously known literature, the tip is expected to have the highest electric potential due to the phenomena of tip-enhanced electric field. Whereas in case of the flat electrode the maximum potential was +1.5V **(****Figure 6D****),** showing the advantage of the MNs shape geometry over the flat geometry in the ability for connecting more analytes on the surface

A real-time on-body trial was performed to compare between the MNs and flat sensor patches **(****Figure 6E-G**). Both sensors were placed on the skin to test the on-line measurement ability **(****Figure 6E-F**), and the data was gathered using a Keithley analyzer. In case of the MNs patch, the response was fast and immediate since the MNs patch directly touched the biomarkers in the ISF, whereas the flat patch gave no response when applied on the skin. The signals and measurements made in these experiments were combined in **Figure 6G****.** As can be seen, the MNs sensor had an immediate response, 10⁸ higher than the flat one.

### Example 5 - Preparation and performance of glucose-, cortisol-, and pH-MN-EGFET Biosensors

In order to test applicability of the MN-EGFET device in the detection of other types of biomarkers, three additional sensors, modified by different biorecognition elements were prepared and examined, said biorecognition elements being: polyaniline (PANI); glucose oxidase; and anti-cortisol antibodies.

pH sensor was fabricated by the deposition of polyaniline (PANI) by cyclic voltammetry (CV) versus SCE electrode from -0.5V to 1.5V for 40 cycles with a scan rate of 100 mV/sec. 0.1 M aniline in HCl was used as deposition solution.

Glucose sensor was fabricated by modifying the Au gate electrode with glucose oxidase (Gox). For this purpose, chitosan was dissolved in 2% (v/v) acetic acid solution by stirring for 1 h to prepare 1% (w/v) chitosan solution; 1 mL of the solution was then mixed with 2 mg of carbon nanotubes and ultrasonicated for 30 min. 30 mg/mL GOx was mixed well with the Chitosan/CNTs solution at a 2:1 (v/v) ratio. A deposition of a mediator layer of Prussian blue on an Au gate was then made by CV versus SCE electrode from -0.2V to 1V for 3 cycles with a scan rate of 100mV/sec. The solution of GOx/Chitosan/CNTs was then drop-casted on the Prussian blue Au gate (5µl) to get the glucose sensor, which was kept at overnight at 4°C until use.

Cortisol sensor was fabricated by modifying the Au gate electrode with anti-cortisol antibodies. For this purpose, The Au gate electrode was first cleaned using ethanol and then with pure water before being thoroughly dried. Immobilization of the monoclonal anti-cortisol was accomplished on the gate by the APTES-GA method. First, the electrode was immersed in 50 mM solution of 6-mercapto-1-hexanol (MCH) in ethanol overnight to get an electrode surface with OH terminals, due to the fact that thiol groups come together to create a self-assembled monolayer (SAM) on the surface of Au. The electrode was washed with ethanol and purified water. It was then immersed in 5% (3-aminopropyl) triethoxysilane (APTES) in a solution of 95% ethanol for 2 h and cleaned with ethanol and water. 2.5% glutaraldehyde (GA) was applied as a crosslinker to the electrode for 1 h and then washed with water. The antibodies (1mg/mL in Tris-HCl Buffer, pH 8) were applied for 2 h. Finally, 100 mM ethanol amine (EA) was added to block the free sites and prevent the nonspecific binding of other molecules.

The sensing performance of each sensor was separately characterized using a Keithley analyzer with corresponding analyte solutions. Furthermore, the effect of confounding biomarkers on the MN-EGFET performance was tested using the sodium ion sensor, the pH sensor, the glucose sensor and the cortisol sensor.

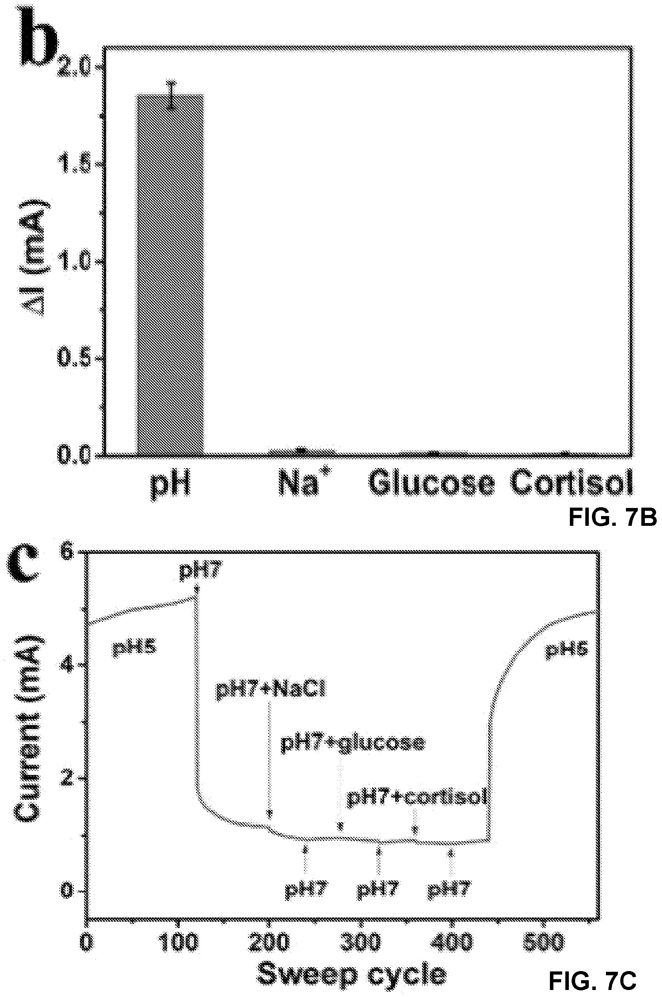

PANI exhibited a unique electrical property in which the resistance can be reversibly changed through the protonation and deprotonation process by acid/base. After coating PANI onto the EG, the MN-EGFET platform had a remarkable sensing performance for pH, with 0.85 mA/pH sensitivity **(****Figure 7A****).** The drain-source current selectively decreased only as the pH increased; meanwhile, only negligible change could be seen when other biomolecules were added, including Na⁺, glucose and cortisol **(****Figure 7B****,** **C****).**

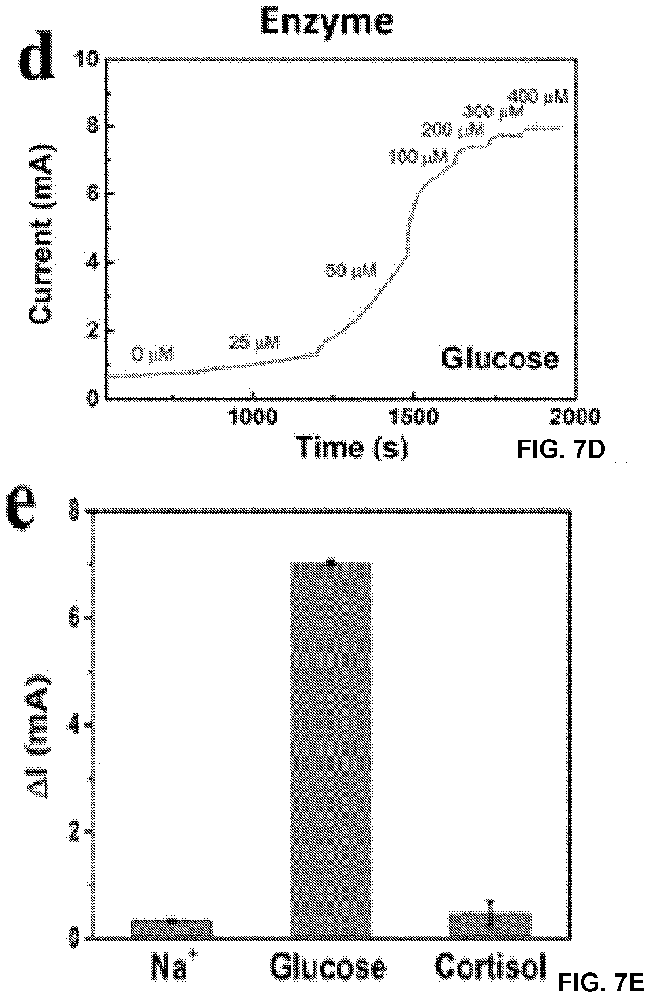

The representative current responses of MN-EGFETs coated with glucose oxidase **(****Figure 7D****)** and anti-cortisol antibodies **(****Figure 7G****)** were measured in 0-400 µM glucose solutions and 0-500 ng/mL cortisol solutions, respectively. For glucose and cortisol, the drain-source current of each constituent was increased as the corresponding analyte concentration increased **(****Figure 7E****,** **H****),** Indicating the selectivity of the appropriate analyte over Na + or pH. Indeed, the glucose sensor and cortisol sensor also respond to pH **(Figure 8F, I).** pH not only affects the activity of the enzyme, but affects the charge of the antibody, so that the current of both glucose and cortisol sensor changed (21% for glucose and 35% for cortisol) when the pH was taken from pH 7 to pH 5. Altogether, these results indicate the ability to control the selectivity of the Na⁺ even under the co-existence of other confounding species in the same ISF source.

### Example 6 - In vitro and in vivo biocompatibility evaluation of the MNs patch

The biocompatibility of the MNs patch for sodium sensing was systematically evaluated both *in vitro* and *in vivo.*

To check the *in vitro* biocompatibility of the MNs patch, mouse pre-osteoblast MC3T3-E1 cells (CRL-2594) and murine fibroblast NIH3T3 cells (CRL-1658), obtained from American Type Culture Collection (ATCC, USA), were cultured with or without the MNs patch for 24 h. MC3T3-E1 cells were cultured in alpha-MEM culture media supplemented with 10% FBS and 1% penicillin-streptomycin at 37°C with incubation in air plus 5% CO₂. NIH3T3 cells were cultured in DMEM culture media supplemented with 10% FBS and 1% penicillin-streptomycin at 37°C with incubation in air plus 5% CO₂.

Cell viability assay, Live/Dead staining, apoptosis assay and reactive oxygen species (ROS) assay for cells cultured with or without MNs patch were carried out separately.

For Cell viability assay, MC3T3-E1 and NIH3T3 cells were cultured in 6-well culture plates (1×10⁵ cells/well) overnight before treatment with microneedles for 24 h. The cells were then washed twice with PBS and were collected for direct number assay in a cell counting chamber according to the following formula: $Cell number / mL = \left(total cell number of the four middle squares/4\right) \times {10}^{4} .$

For Live/Dead staining assay, MC3T3-E1 and NIH3T3 cells were cultured in 6-well culture plates (1×10⁵ cells/well) overnight before being treated with microneedles for 24 h. The cells were then washed twice with PBS and stained with the Live/Dead TM Cell Imaging Kit for 30 min. The cells were washed 3 times with PBS and the living cells or dead cells were photographed by fluorescence microscopy (Nikon ECLIPSE Ti-U, Japan).

For Apoptosis assays, MC3T3-E1 and NIH3T3 cells were cultured in 6-well culture plates (1×10⁵ cells/well) overnight before being treated with the microneedles for 24 h. The cells were twice washed with PBS and stained with Annexin V-FITC for 15 min and PI staining solution for 10 min. After stimulation, the apoptotic cells were analyzed by flow cytometry (BD Biosciences).

For ROS staining assay, cellular ROS was detected by DCFH-DA fluorescence probe. MC3T3-E1 and NIH3T3 cells were cultured in 6-well culture plates (1×10⁵ cells/well) overnight before treating with the microneedles for 24 h. The cells were then washed twice with PBS and stained with 10 µM DCFH-DA for 30 min. They were observed and photographed in a fluorescent microscope as above.

There were no statistically significant differences (n=3 biological replicates) in both MC3T3-E1 and NIH3T3 cells after 24 h culturing with or without the MNs patch, indicating that the patch did not significantly affect cell viability **(****Figure 8A****,** **B****).**

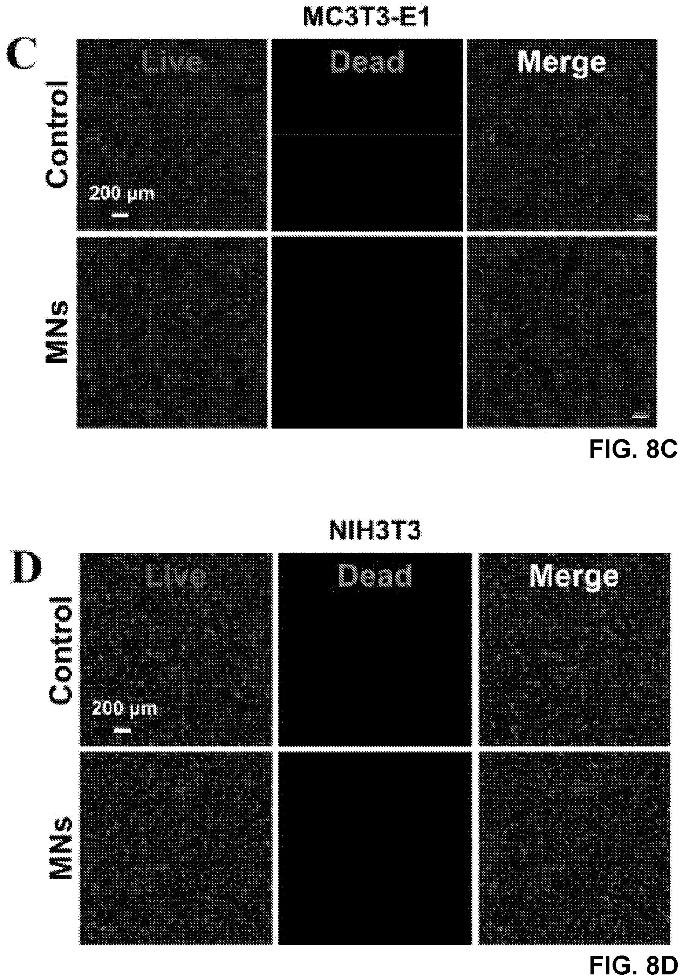

Furthermore, Live/Dead staining of MC3T3-E1 **(****Figure 8C****)** and NIH3T3 **(****Figure 8D****)** show that there are living cells, with only a small number of cells dying after 24 h both with and without MNs patches.

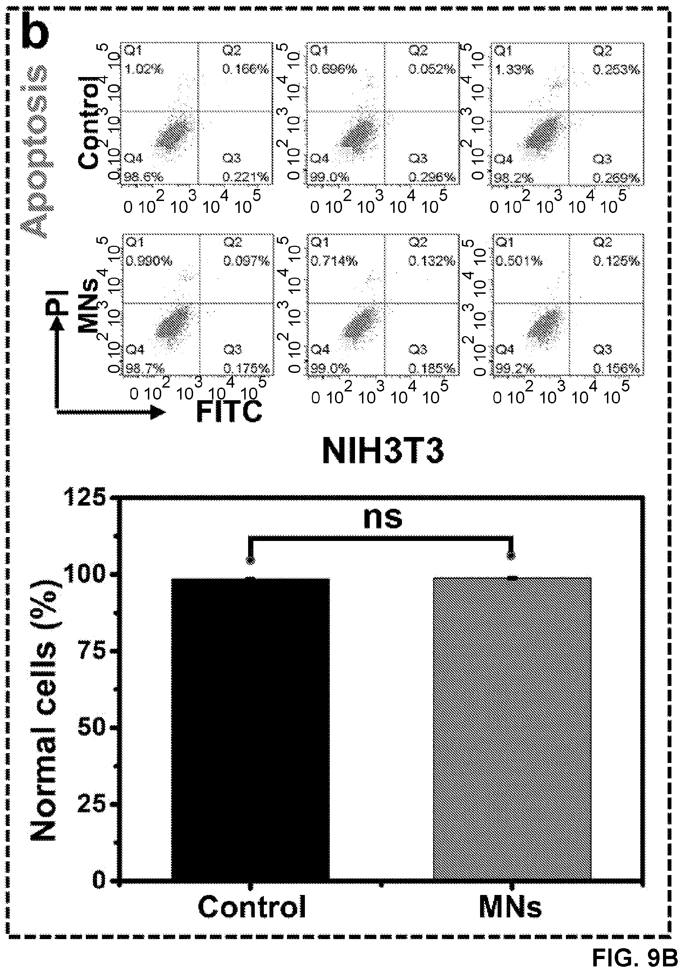

Further analysis of apoptosis assays indicated that there are almost 98% live cells both in MC3T3-E1 **(****Figure 9a****)** and NIH3T3 **(****Figure 9b****)** after 24 hours of culturing with the MNs patch, which are consistent with the results of the one after 24 hours of culturing without the MNs patch.

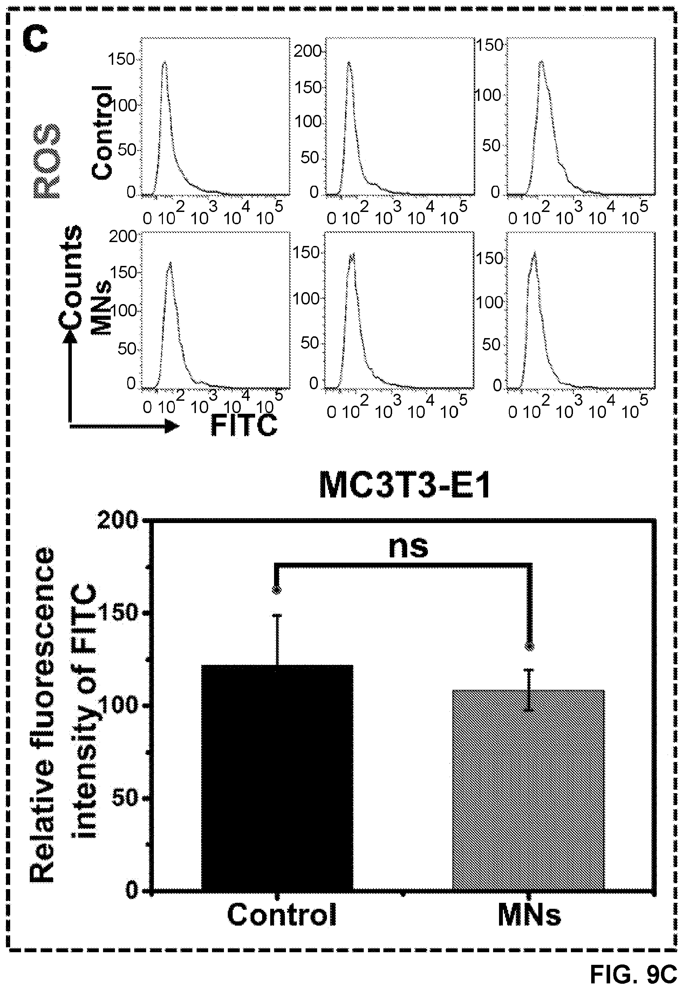

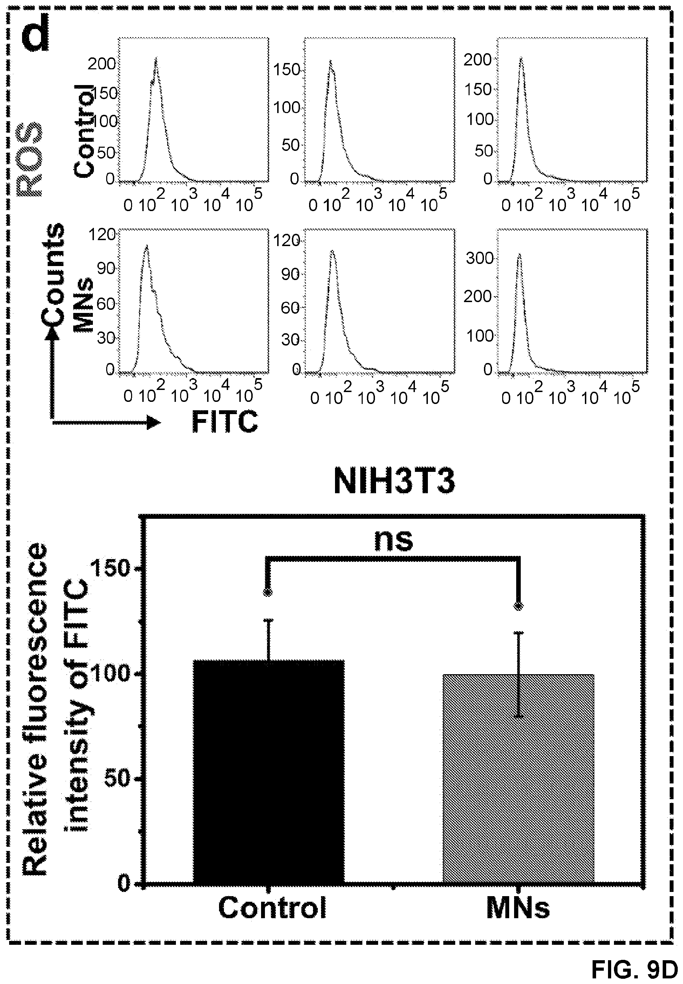

Analysis of ROS assays has shown no statistically significant differences (n=3 biological replicates), both in MC3T3-E1 **(****Figure 9c****)** and NIH3T3 **(****Figure 9d****)** after 24 hours of culturing with/without the MNs patch.

Together these results indicate that the proposed MNs patch has no significant impact on the MC3T3-E1 and NIH3T3 cells viability, demonstrating their excellent *in vitro* biocompatibility.

To further evaluate the biocompatibility of the MNs patch, an *in vivo* study involved an animal model was conducted. For this purpose, hematoxylin and eosin (H&E) staining of the organs, together with CD3 immunofluorescent staining assay of skin tissue were carried out.

For H&E staining, healthy 4-week female BALB/c mice were purchased from an animal laboratory center of Guangdong province and housed in a SPF laboratory animal room. MNs patches were inserted into the back skin of the mice by a thumb press and removed after different insertion times (control, 1 h, 1 day, 3 days, and 7 days). The major organs (including heart, liver, spleen, lung, and kidney) were resected to assess toxicity.

For CD3 Immunofluorescent staining assay of skin tissue, healthy 4-week female BALB/c mice were treated with the microneedles for different times and euthanized so that the skin could be separated and embedded into OCT agent prior to being sectioned at 5 µm for further immunofluorescent staining. Frozen sections were treated with 4% paraformaldehyde and 0.3% Triton X-100 solution. The frozen sections were blocked with 2% BSA solution for 30 min and incubated with anti-CD3 antibody (ab135372) overnight. On the second day, the frozen sections were washed with 1× TBST for 3 times and incubated with goat anti-rabbit IgG Alexa Fluor 568 (A11011, Thermo Fisher) for 60 min. The frozen sections were treated with antifade mounting medium with DAPI (H-1200) and imaged with a confocal microscope (Nikon A1R, Japan).

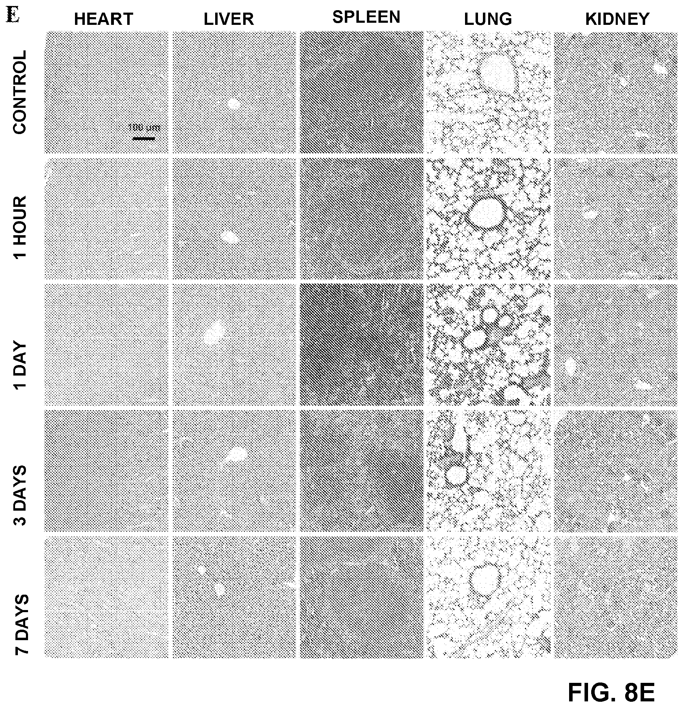

No apparent pathological changes and toxic effects were seen in the organs within the different insertion times **(****Figure 8E****),** indicating that the MNs patch were not harmful.

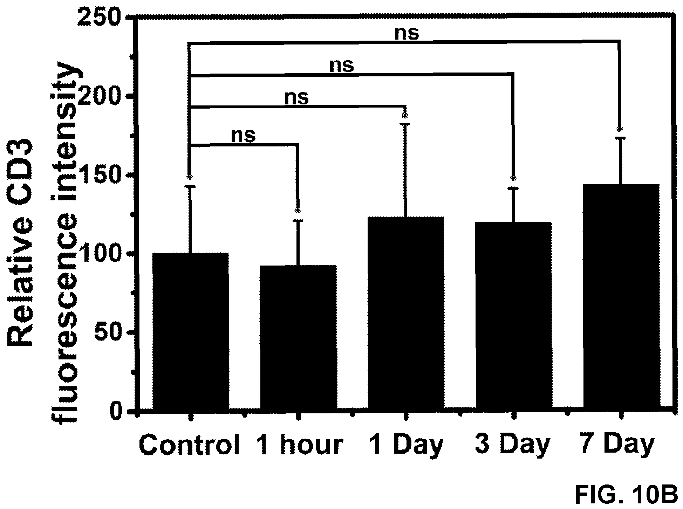

CD3 is highly expressed in T cells, which are important in the inflammatory response. CD3 immunofluorescent staining of skin tissue from each group after different insertion times also indicated no significant differences between the MNs patch-inserted groups with different inserting time and the control group **(****Figure 10****).** These results show the MNs patch had no significant impact on the major mice organs and immuno-inflammatory responses, and thus the MNs patch has high potential regarding its *in vivo* biocompatibility.

### Example 7 - Biocompatibility of the skin-MN interface

To evaluate the recovery of the skin following peeling-off, healthy 4-week female BALB/c mice were treated with the microneedles. The micro-holes on the mice were photographed after microneedle insertion and removal. To validate that the MNs do reach as far as the ISF, MNs-treated mice were euthanized and skin samples were collected for H&E assay. H&E images showed that the MNs reached the dermis layer, but not the subcutaneous tissues, demonstrating that MNs had reached the ISF. As is shown in **Figure 10A-B**, micro-holes appeared on mice skin after removing the MNs. However, the micro-holes disappeared completely within 30 min after the MNs removal, and the skin quickly recovered, indicating that MNs do not damage the skin **(Figure 10C).** Furthermore, the skin did not show erythema or oedema 30 min after MNs removal **(Figure 10D),** i.e., there was no inflammatory cell infiltration or pathophysiological response of skin to applying and removing MNs.

### Example 8 - Demonstration of the wearable sodium sensing MNs patch for on-body trial

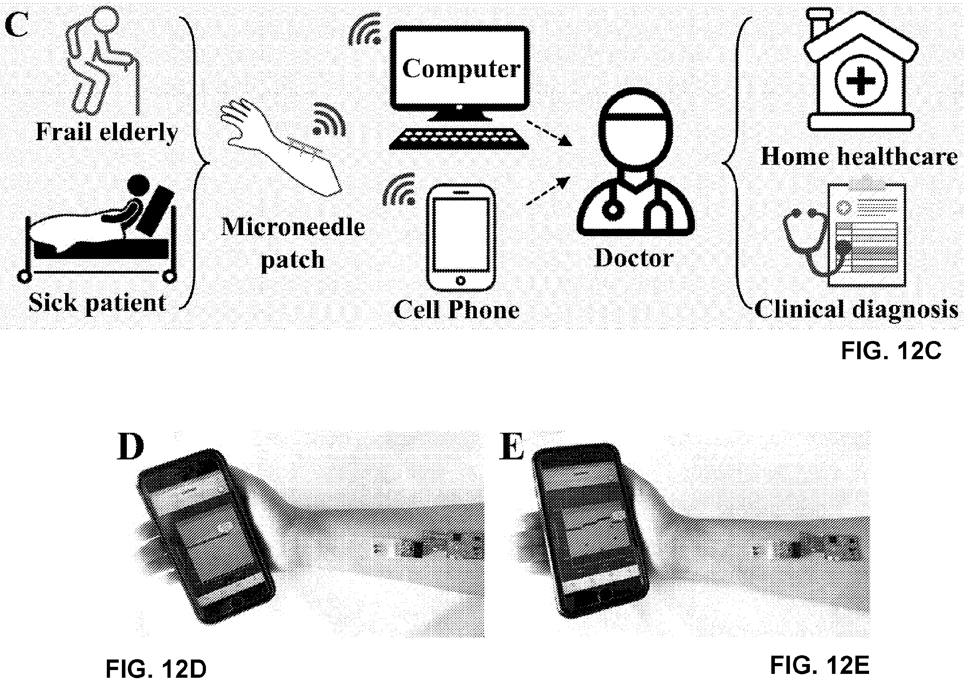

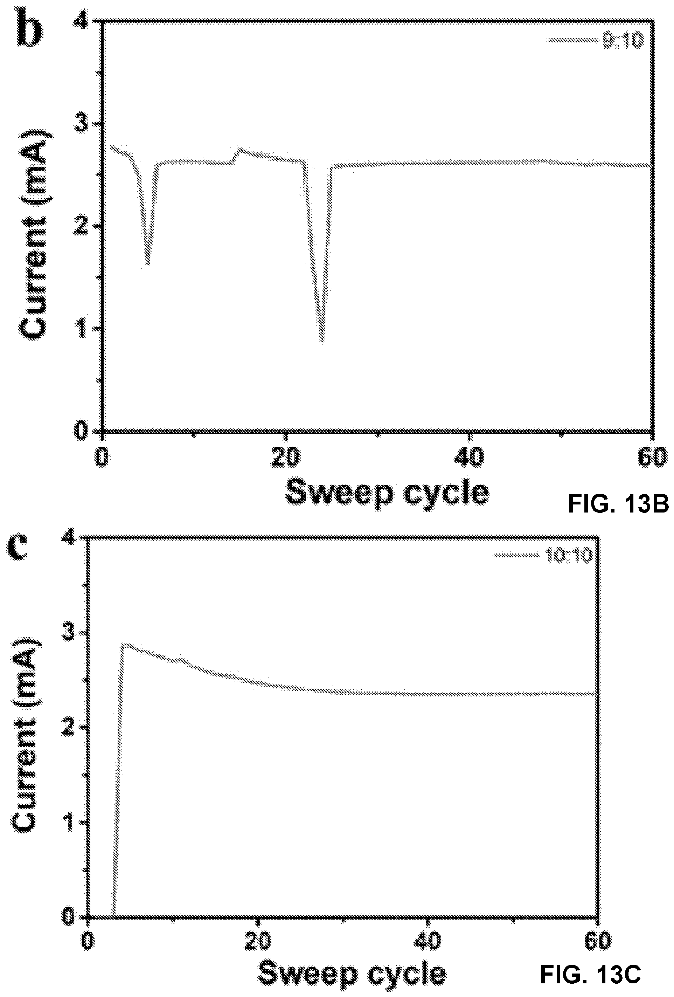

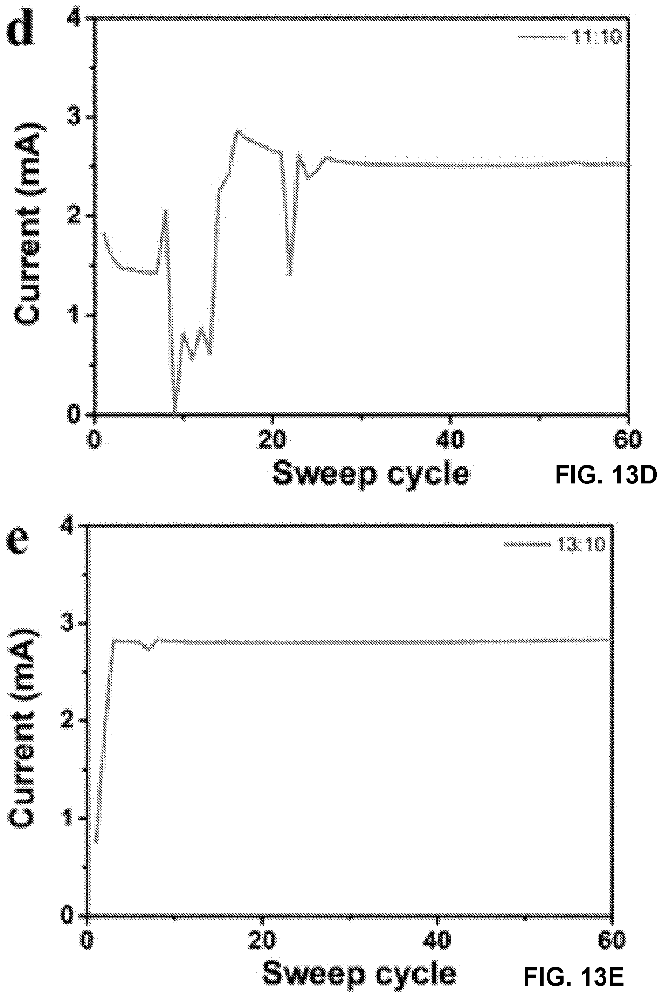

To evaluate the long-term health monitoring performance of the wearable MN-EGFET patch, Na⁺ MN-EGFET sensor was used to take on-body measurements for several hours of a healthy individual **(****Figure 12** and **Figure 13****).**

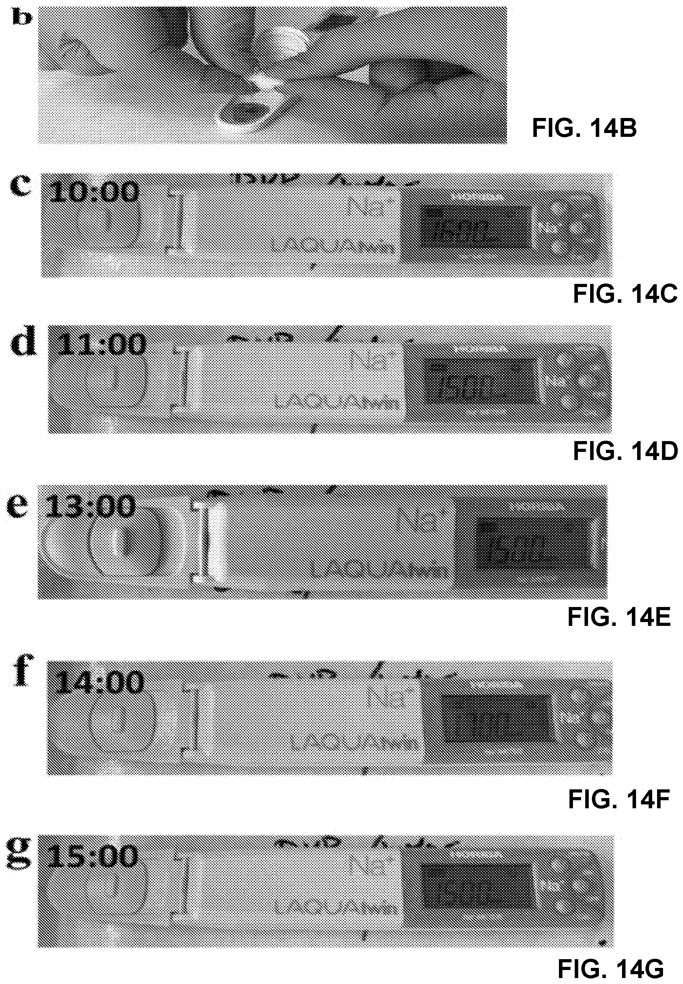

As a reference, sweat was collected after exercise and examined by a commercial device (Na⁺ meter, HORIBA, B-722) **(****Figure 14****).** As is shown at **Figure 12B****,** sodium concentration fluctuation in sweat showed a similar tendency to that in the ISF, but with a time delay since the sweat is derived from the ISF through sweat glands, so that it has a similar sodium concentration fluctuation tendency with ~40 min delay.

The proposed MN-patch can collect health status information of an individual without the need to exercise for sweating, which makes it even more convenient and practical for real-life applications, especially for very sick patients and frail elderly people. With the help of wireless data communication and IoT technology, frail elderly and sick patients' health status can be measured through biosensors and uploaded onto the IoT cloud by computer/smartphone **(****Figure 12C****).** Doctors can readily get the information about the patient and give professional diagnosis without the need for face-to-face consultation, which is more effective and efficient in many cases.

To evaluate the applicability of wireless health monitoring with the developed device, the MN-EGFET patch was integrated with the wireless Bluetooth transmitter (ecFlex, Zimmer and Peacock) by using an additionally designed circuit **(****Figure 15** -**Figure 15a** **shows circuit diagram and** **Figure 15b** **shows exemplary built circuit).** In some examples, the circuit comprises a plurality of resistive elements (e.g. resistors) R1, R2, R3 and R4. In some examples, the source is coupled to a common potential via resistive element R1. In some examples, the drain is coupled to a respective terminal of a transmitter, such as a wireless Bluetooth transmitter. In some examples, the reference electrode is coupled to a predetermined potential, via resistive element R2. In some examples, the reference electrode is further coupled to the common potential via resistive elements R3 and R4, R3 and R4 being coupled in parallel. Although figure 15 shows exemplary resistance values for R1 - R4, this is not meant to be limiting in any way.

To mimic a human body's Na⁺ concentration fluctuation, we dropped NaCl solutions onto the Na⁺ sensor in the following order: 10mM-40mM-160mM-10mM. The concentration change was continually recorded, successfully indicating the sodium change just using a smartphone **(****Figure. 12D-F**).

It is appreciated by persons skilled in the art that the present disclosure is not limited by what has been particularly shown and described hereinabove. Rather the scope of the present disclosure includes both combinations and sub-combinations of various features described hereinabove as well as variations and modifications. Therefore, the disclosure is not to be constructed as restricted to the particularly described examples, and the scope and concept of the disclosure will be more readily understood by references to the claims, which follow.

## Claims

1. A wearable extended gate field effect transistor (EGFET) device for detecting and/or measuring a constituent of an interstitial fluid from the skin of a subject, the device comprising:
(a) a polymeric substrate for fixing to the skin of the subject;
(b) an extended gate electrode comprising a first microneedle configured for accessing the interstitial fluid and a first electric contact, wherein the first microneedle and the first electric contact are disposed on the polymeric substrate;
(c) a reference electrode comprising a second electric contact, wherein the second electric contact is disposed on the polymeric substrate; and
(d) a field effect transistor (FET) associated with the first microneedle through the first electric contact;
**characterized in that** the reference electrode further comprises a second microneedle, being disposed on the polymeric substrate;
wherein a portion of the polymeric substrate on which the first electric contact is disposed is stretchable.

2. The device according to claim 1, wherein the second electric contact is disposed on the stretchable portion of the polymeric substrate, preferably wherein the first microneedle is solid, and more preferably wherein the second microneedle is solid.

3. The device according to claim 1 or 2, wherein the polymeric substrate has a thickness gradient between the first microneedle and the first electric contact; or wherein the thickness of a portion of the polymeric substrate on which the first microneedle is disposed is at least 50% higher than the thickness of a portion of the polymeric substrate on which the first electric contact is disposed, preferably wherein the portion of the polymeric substrate on which the first microneedle is disposed is substantially rigid and the stretchable portion of the polymeric substrate on which the first electric contact is disposed is flexible, or wherein the portion of the polymeric substrate on which the first microneedle is disposed and the portion of the polymeric substrate on which the first electric contact is disposed are made of the same polymeric material.

4. The device according to any one of claims 1 to 3, wherein the polymeric substrate is made of a polymeric material selected from the group consisting of styrene-block-isoprene-block styrene (SIS), 1-styrene-butadiene-styrene block copolymer (SBS), 2-styrene ethylene butylene styrene block copolymer (SEBS), polydimethylsiloxane (PDMS), polybutadiene rubber, polyurethane thermoplastic elastomer, low-density polyethylene
(LDPH), polyisoprene, chloroprene rubber (CR), silicone rubber, and combinations and derivatives thereof; or wherein the first microneedle, the second microneedle or both are made of a material selected from the group consisting of a polymer, metal, metal alloy, carbon, and combinations thereof, preferably wherein the polymer is selected from the group consisting of polyester, polystyrene, polycarbonate, poly (methyl methacrylate), acrylate, polyvinylpyrrolidone, epoxy-based negative photoresist, and combinations thereof, more preferably wherein the first microneedle is made of polystyrene, coated by a metal selected from Au, Pt, and Ni.

5. The device according to claim 4, wherein the first microneedle is modified with a biorecognition element selected from the group consisting of an enzyme, antibody, aptamer, ion-selective membrane (ISM), protonically doped polymer, DNA, ABTS (2,2'-azino-bis(3-ethylbenzothiazoline-6-sulfonic acid)), molecularly imprinted polymer (MIP), and combinations thereof, preferably wherein the biorecognition element is bound to the first microneedle via a linker or is held within a supporting film or matrix.

6. The device according to claim 5, wherein said constituent is a sodium ion and the biorecognition element comprises Na ionophore X and sodium tetrakis[3,5-bis(trifluoromethyl)phenyl] borate (Na-TFPB), preferably wherein the biorecognition material is immobilized on the first microneedle by polyvinyl chloride (PVC) and bis(2-ethylehexyl) sebacate (DOS), more preferably wherein said constituent is cortisol and the biorecognition element comprises monoclonal anti-cortisol, and most preferably wherein the biorecognition element is immobilized on the first microneedle via a (3-aminopropyl)triethoxysilane (APTES) and glutaraldehyde linker; or wherein said constituent is glucose and the biorecognition element is glucose oxidase, preferably wherein the biorecognition element is immobilized on the first microneedle by chitosan, wherein chitosan is mixed with carbon nanotubes; or wherein said constituent is a hydronium ion and the biorecognition element is polyaniline (PANI).

7. The device according to claim 4, wherein the second microneedle is made of polystyrene coated by Ag, preferably wherein the second microneedle is modified with a metal material configured to apply voltage on the surface of the second microneedle, the gate voltage of the FET being responsive to the metal material, more preferably wherein the metal material is selected from the group consisting of Ag/AgCl/PVB and Au.

8. The device according to any one of claims 1 to 7, wherein the first microneedle, the second microneedle or both have a conical shape having a height between about 250 µm and about 5 mm and a diameter at its base between about 100 µm and 2.5 mm; or wherein the extended gate electrode comprises a plurality of first microneedles disposed on the polymeric substrate and arranged in an array, wherein the FET is associated with the plurality of first microneedles through the first electric contact; or wherein the reference electrode comprises a plurality of second microneedles disposed on the polymeric substrate and arranged in an array; or wherein the first electric contact, the second electric contact or both comprise electrically conductive elongated nanostructures, preferably wherein the electrically conductive elongated nanostructures are selected from the group consisting of nanotubes, nanowires, nanoribbons, nano-whiskers, nanostrips, nanorods, and combinations thereof, more preferably wherein the electrically conductive elongated nanostructures are made of a material selected from the group consisting of a metal, metal alloy, carbon, and combinations thereof, and most preferably wherein the first electric contact and the second electric contact comprises silver nanowires (AgNWs).

9. The device according to any one of claims 1 to 8, wherein the FET is selected from the group consisting of a metal-oxide-semiconductor field-effect transistor (MOSFET), junctionless nanowire transistor (JLNT), metal-nitride-oxide-semiconductor transistor (MNOS), junction field-effect transistor (JFET), static induction transistor (SIT); heterostructure insulated-gate field-effect transistor (HIGFET), modulation-doped field-effect transistor (MODFET); tunnel field-effect transistor (TFET), high-electron-mobility transistor (HEMT), metal-semiconductor field-effect transistor (MESFET), nanoparticle organic memory field-effect transistor (NOMFET), graphene nanoribbon field-effect transistor (GNRFET), vertical-slit field-effect transistor (VeSFET), carbon nanotube field-effect transistor (CNTFET), organic field-effect transistor (OFET), quantum field effect transistor (QFET), Schottky-barrier field-effect transistor (SB-FET), and graphene-based field effect transistor (GFET), preferably wherein the FET is a MOSFET.

10. A system for detecting and/or measuring a constituent of an interstitial fluid from the skin of a subject, the system comprising:
the wearable EGFET device according to any one of claims 1 to 9; and
at least one of:
a control unit being in electrical communication with the EGFET device, which measures an electrical signal generated by the FET in response to an interaction between the first microneedle and said constituent; and
a transmitter, which receives the electrical signal generated by the FET in response to the interaction between the first microneedle and said constituent and transmits said signal to a remote server and/or to a portable electronic device.

11. The system according to claim 10, wherein the control unit is in electrical communication with each one of the FET, extended gate electrode, and reference electrode within the EGFET device; or wherein the system further comprises a display unit in electrical communication with the control unit for displaying information related to the measuring of the electrical signal, and/or means for determining concentration of the constituent in the interstitial fluid upon receipt of the electrical signal.

12. A method for detecting and/or measuring a constituent of an interstitial fluid from the skin of a subject, the method comprising:
(a) providing the system of any one of claims 10 to 11;
(b) fixing the wearable EGFET to the skin of a subject, thereby enabling an interaction between the first microneedle and said constituent;
(c) measuring an electrical signal generated by the FET in response to the interaction between the first microneedle and said constituent; and
(d) analyzing the electrical signal by at least one of the control unit, the remote server and the portable electronic device.

13. The method according to claim 12, wherein fixing the wearable EGFET to the skin of the subject comprises directly contacting the first microneedle with the skin of the subject, and wherein the measuring step is performed while the wearable EGFET is fixed to the skin of the subject; or wherein analyzing the electrical signal comprises comparing the electrical signal with a calibration curve and/or reference data; or wherein the method further comprises displaying information related to the measuring of the electrical signal or a result of the step of analyzing (step (d)) onto a display unit being in electrical communication with the control unit, the remote server, or the portable electronic device.

14. A method for fabricating the wearable EGFET device according to any one of claims 1 to 9, the method comprising:
(a) providing the first microneedle, the first electric contact, and the polymeric substrate;
(b) providing the FET;
(c) connecting the first electric contact with the polymeric substrate;
(d) connecting the first microneedle with the polymeric substrate; and
(e) associating the FET with the first microneedle.

15. The method according to claim 14, wherein steps (a), and (b) are performed in any order or wherein steps (a), and (b) are performed simultaneously, or wherein the method further comprises a step of providing the second microneedle and the second electric contact, and a step of connecting the second microneedle and the second electric contact with the polymeric substrate, preferably wherein providing the first electric contact and/or providing the second electric contact comprises spray-coating hydrophobic electrically conductive elongated nanostructures onto a Si wafer coated with a mask having a predefined opening, peeling the mask and annealing the obtained first electric contact and/or second electric contact, or wherein providing the polymeric substrate comprises forming a first substantially flexible and stretchable polymeric film and combining said film with a second substantially flexible and stretchable polymeric film, which has been pre-stretched, or wherein providing the polymeric substrate comprises thickening a portion of the polymeric substrate by applying a solution of the substantially flexible and stretchable polymer onto said portion, preferably wherein the step of connecting the first electric contact with the polymeric substrate comprises spin-coating a solution of the substantially flexible and stretchable polymer onto the first electric contact to form the first substantially flexible and stretchable polymeric film prior to combining said first substantially flexible and stretchable polymeric film with the second substantially flexible and stretchable polymeric film, which has been pre-stretched, or wherein the step of connecting the first microneedle with the polymeric substrate comprises fixing the first microneedle onto the thickened portion of the polymeric substrate.

## Patentansprüche

1. Tragbares Feldeffekttransistorgerät mit verlängertem Gatter (EGFET) zum Erfassen und/oder Messen eines Bestandteils der Interstitialflüssigkeit aus der Haut einer Person, worin das Gerät umfasst:
(a) ein Polymersubstrat zum Befestigen an der Haut der Person;
(b) eine verlängerte Gatter-Elektrode, die eine erste Mikronadel, die für den Zugang zur Interstitialflüssigkeit ausgelegt ist, und einen ersten elektrischen Kontakt umfasst, worin die erste Mikronadel und der erste elektrische Kontakt auf dem Polymersubstrat angeordnet sind;
(c) eine Referenzelektrode, die einen zweiten elektrischen Kontakt umfasst, worin der zweite elektrische Kontakt auf dem Polymersubstrat angeordnet ist; und
(d) einen Feldeffekttransistor (FET) der über den ersten elektrischen Kontakt mit der ersten Mikronadel verbunden ist;
**dadurch gekennzeichnet, dass** die Referenzelektrode ferner eine zweite Mikronadel umfasst, die auf dem Polymersubstrat angeordnet ist;
worin ein Abschnitt des Polymersubstrats, auf dem der erste elektrische Kontakt angeordnet ist, dehnbar ist.

2. Vorrichtung nach Anspruch 1, worin der zweite elektrische Kontakt auf dem dehnbaren Abschnitt des Polymersubstrats angeordnet ist, vorzugsweise worin die erste Mikronadel massiv ist, und noch bevorzugter worin die zweite Mikronadel massiv ist.

3. Vorrichtung nach Anspruch 1 oder 2, worin das Polymersubstrat einen Dickengradienten zwischen der ersten Mikronadel und dem ersten elektrischen Kontakt aufweist; oder worin die Dicke eines Abschnitts des Polymersubstrats, auf dem die erste Mikronadel angeordnet ist, mindestens 50% größer ist als die Dicke eines Abschnitts des Polymersubstrats, auf dem der erste elektrische Kontakt angeordnet ist, vorzugsweise worin der Abschnitt des Polymersubstrats, auf dem die erste Mikronadel angeordnet ist, im Wesentlichen starr ist und der dehnbare Abschnitt des Polymersubstrats, auf dem der erste elektrische Kontakt angeordnet ist, flexibel ist, oder worin der Abschnitt des Polymersubstrats, auf dem die erste Mikronadel angeordnet ist, und der Abschnitt des Polymersubstrats, auf dem der erste elektrische Kontakt angeordnet ist, aus demselben Polymermaterial bestehen.

4. Vorrichtung nach einem der Ansprüche 1 bis 3, worin das Polymersubstrat aus einem Polymermaterial besteht, ausgewählt aus der Gruppe bestehend aus Styrol-Block-Isopren-Block-Styrol (SIS), 1-Styrol-Butadien-Styrol-Blockcopolymer (SBS), 2-Styrol-Ethylen-Butylen-Styrol-Blockcopolymer (SEBS), Polydimethylsiloxan (PDMS), Polybutadien-Kautschuk, thermoplastischem Polyurethan-Elastomer, Polyethylen niedriger Dichte (LDPH), Polyisopren, Chloropren-Kautschuk (CR), Silikonkautschuk sowie Kombinationen und Derivate davon; oder worin die erste Mikronadel, die zweite Mikronadel oder beide aus einem Material bestehen, ausgewählt aus der Gruppe bestehend aus einem Polymer, einem Metall, einer Metalllegierung, Kohlenstoff und Kombinationen davon, worin das Polymer vorzugsweise ausgewählt aus der Gruppe bestehend aus Polyester, Polystyrol, Polycarbonat, Poly(methylmethacrylat), Acrylat, Polyvinylpyrrolidon, epoxidbasiertem Negativ-Fotolack und Kombinationen davon, worin die erste Mikronadel vorzugsweise aus Polystyrol besteht, das mit einem Metall beschichtet ist ausgewählt unter Au, Pt und Ni.

5. Vorrichtung nach Anspruch 4, worin die erste Mikronadel mit einem Bioerkennungselement modifiziert ist, ausgewählt aus der Gruppe bestehend aus einem Enzym, einem Antikörper, einem Aptamer, einer ionenselektiven Membran (ISM), einem protonisch dotierten Polymer, DNA, ABTS (2,2'-Azino-bis(3-ethylbenzothiazolin-6-sulfonsäure)), einem molekular geprägtem Polymer (MIP) und Kombinationen davon, worin das Bioerkennungselement vorzugsweise über einen Linker an die erste Mikronadel gebunden ist oder in einer Trägerfolie oder einer Matrix gehalten wird.

6. Vorrichtung nach Anspruch 5, worin der Bestandteil ein Natriumion ist und das Bioerkennungselement Na-Ionophor X und Natriumtetrakis[3,5-bis(trifluormethyl)phenyl]borat (Na-TFPB) umfasst, vorzugsweise worin das Bioerkennungsmaterial durch Polyvinylchlorid (PVC) und Bis(2-ethylehexyl)sebacat (DOS) auf der ersten Mikronadel immobilisiert ist, noch bevorzugter, worin der Bestandteil Cortisol ist und das Bioerkennungselement monoklonale Anti-Cortisol-Antikörper umfasst, und am meisten bevorzugt, worin das Bioerkennungselement über einen (3-Aminopropyl)triethoxysilan (APTES)- und Glutaraldehyd-Linker auf der ersten Mikronadel immobilisiert ist; oder worin der Bestandteil Glukose ist und das Bioerkennungselement Glukoseoxidase ist, vorzugsweise worin das Bioerkennungselement mittels Chitosan auf der ersten Mikronadel immobilisiert ist, worin das Chitosan mit Kohlenstoffnanoröhren gemischt ist; oder worin der Bestandteil ein Hydroniumion ist und das Bioerkennungselement Polyanilin (PANI) ist.

7. Vorrichtung nach Anspruch 4, worin die zweite Mikronadel aus mit Ag beschichtetem Polystyrol besteht, vorzugsweise worin die zweite Mikronadel mit einem Metallmaterial modifiziert ist, das ausgestaltet ist, eine Spannung an die Oberfläche der zweiten Mikronadel anzulegen, worin die Gatter-Spannung des FET auf das Metallmaterial anspricht, noch bevorzugter worin das Metallmaterial ausgewählt ist aus der Gruppe bestehend aus Ag/AgCl/PVB und Au.

8. Vorrichtung nach einem der Ansprüche 1 bis 7, worin die erste Mikronadel, die zweite Mikronadel oder beide eine konische Form mit einer Höhe zwischen etwa 250 µm und etwa 5 mm und einem Durchmesser an ihrer Basis zwischen etwa 100 µm und 2,5 mm aufweisen; oder worin die verlängerte Gatter-Elektrode mehrere erste Mikronadeln umfasst, die auf dem Polymersubstrat angeordnet und in einer Anordnung angeordnet sind, worin der FET über den ersten elektrischen Kontakt mit den mehreren ersten Mikronadeln verbunden ist; oder worin die Referenzelektrode mehrere zweite Mikronadeln umfasst, die auf dem Polymersubstrat angeordnet und in einer Anordnung angeordnet sind; oder worin der erste elektrische Kontakt, der zweite elektrische Kontakt oder beide elektrisch leitfähige, längliche Nanostrukturen umfassen, vorzugsweise worin die elektrisch leitfähigen, länglichen Nanostrukturen ausgewählt sind aus der Gruppe bestehend aus Nanoröhren, Nanodrähten, Nanobändern, Nanowhiskern, Nanostreifen, Nanostäbchen und Kombinationen davon, worin die elektrisch leitfähigen, länglichen Nanostrukturen vorzugsweise aus einem Material bestehen, ausgewählt aus der Gruppe bestehend aus einem Metall, einer Metalllegierung, Kohlenstoff und Kombinationen davon, und worin der erste elektrische Kontakt und der zweite elektrische Kontakt am bevorzugtesten Silbernanodrähte (AgNW) umfassen.

9. Vorrichtung nach einem der Ansprüche 1 bis 8, worin der FET aus der Gruppe ausgewählt ist, bestehend aus einem Metall-Oxid-Halbleiter-Feldeffekttransistor (MOSFET), einem übergangslosen Nanodrahttransistor (JLNT), einem Metall-Nitrid-Oxid-Halbleiter-Transistor (MNOS), einem Übergangs-Feldeffekttransistor (JFET) einem statischen Induktionstransistor (SIT), einem Heterostruktur-Feldeffekttransistor mit isoliertem Gatter (HIGFET), einem modulationsdotierten Feldeffekttransistor (MODFET), einem Tunnel-Feldeffekttransistor (TFET), einem Transistor mit hoher Elektronenbeweglichkeit (HEMT), einem Metall-Halbleiter-Feldeffekttransistor (MESFET), einem Nanopartikel-Organik-Speicher-Feldeffekttransistor (NOMFET), einem Graphen-Nanoband-Feldeffekttransistor (GNRFET), einem Vertikal-Schlitz-Feldeffekttransistor (VeSFET), einem Kohlenstoffnanoröhren-Feldeffekttransistor (CNTFET), einem organischen Feldeffekttransistor (OFET), einem Quanten-Feldeffekttransistor (QFET), einem Schottky-Barriere-Feldeffekttransistor (SB-FET) und einem Feldeffekttransistor auf Graphenbasis (GFET), vorzugsweise worin der FET ein MOSFET ist.

10. System zum Erfassen und/oder Messen eines Bestandteils einer Interstitialflüssigkeit aus der Haut einer Person, worin das System umfasst:
die tragbare EGFET-Vorrichtung gemäß einem der Ansprüche 1 bis 9; und
mindestens eines der folgenden Elemente:
eine Steuereinheit, die in elektrischer Verbindung mit der EGFET-Vorrichtung steht und ein elektrisches Signal erfasst, das vom FET als Reaktion auf eine Wechselwirkung zwischen der ersten Mikronadel und dem genannten Bestandteil erzeugt wird; und
einen Sender, der das vom FET als Reaktion auf die Wechselwirkung zwischen der ersten Mikronadel und dem genannten Bestandteil erzeugte elektrische Signal empfängt und dieses Signal an einen Remote-Server und/oder an ein tragbares elektronisches Gerät überträgt.

11. System nach Anspruch 10, worin die Steuereinheit mit dem FET, der verlängerten Gatter-Elektrode und der Referenzelektrode innerhalb der EGFET-Vorrichtung jeweils in elektrischer Verbindung steht; oder worin das System ferner eine Anzeigeeinheit umfasst, die mit der Steuereinheit in elektrischer Verbindung steht, um Informationen bezüglich der Messung des elektrischen Signals anzuzeigen, und/oder Mittel zur Bestimmung der Konzentration des Bestandteils in der Interstitialflüssigkeit nach Empfang des elektrischen Signals.

12. Verfahren zum Nachweis und/oder zur Messung eines Bestandteils einer interstitiellen Flüssigkeit aus der Haut einer Person, wobei das Verfahren umfasst:
(a) Bereitstellen des Systems gemäß einem der Ansprüche 10 bis 11;
(b) Befestigen des tragbaren EGFET an der Haut eines Probanden, wodurch eine Wechselwirkung zwischen der ersten Mikronadel und dem genannten Bestandteil ermöglicht wird;
(c) Messen eines elektrischen Signals, das vom FET als Reaktion auf die Wechselwirkung zwischen der ersten Mikronadel und dem genannten Bestandteil erzeugt wird; und
(d) Analysieren des elektrischen Signals durch mindestens eine der folgenden Einheiten: die Steuereinheit, den Remote-Server und das tragbare elektronische Gerät.

13. Verfahren nach Anspruch 12, wobei das Befestigen des tragbaren EGFET an der Haut der Testperson das direkte Inkontaktbringen der ersten Mikronadel mit der Haut der Testperson umfasst und wobei der Messschritt durchgeführt wird, während der tragbare EGFET an der Haut der Testperson befestigt ist; oder wobei das Analysieren des elektrischen Signals das Vergleichen des elektrischen Signals mit einer Kalibrierungskurve und/oder Referenzdaten umfasst; oder wobei das Verfahren ferner das Anzeigen von Informationen bezüglich der Messung des elektrischen Signals oder eines Ergebnisses des Analyseschritts (Schritt (d)) auf einer Anzeigeeinheit umfasst, die in elektrischer Verbindung mit der Steuereinheit, dem Remote-Server oder dem tragbaren elektronischen Gerät steht.

14. Verfahren zur Herstellung der tragbaren EGFET-Vorrichtung gemäß einem der Ansprüche 1 bis 9, wobei das Verfahren umfasst:
(a) Bereitstellen der ersten Mikronadel, des ersten elektrischen Kontakts und des Polymersubstrats;
(b) Bereitstellen des FET;
(c) Verbinden des ersten elektrischen Kontakts mit dem Polymersubstrat;
(d) Verbinden der ersten Mikronadel mit dem Polymersubstrat; und
(e) Verbinden des FET mit der ersten Mikronadel.

15. Verfahren nach Anspruch 14, wobei die Schritte (a) und (b) in beliebiger Reihenfolge oder gleichzeitig durchgeführt werden oder wobei das Verfahren ferner einen Schritt zum Bereitstellen der zweiten Mikronadel und des zweiten elektrischen Kontakts umfasst, sowie einen Schritt zum Verbinden der zweiten Mikronadel und des zweiten elektrischen Kontakts mit dem Polymersubstrat, vorzugsweise wobei das Bereitstellen des ersten elektrischen Kontakts und/oder das Bereitstellen des zweiten elektrischen Kontakts das Aufsprühen hydrophober, elektrisch leitfähiger, länglicher Nanostrukturen auf einen Si-Wafer umfasst, der mit einer Maske mit einer vordefinierten Öffnung beschichtet ist, das Abziehen der Maske und das Tempern des erhaltenen ersten elektrischen Kontakts und/oder des zweiten elektrischen Kontakts, oder wobei das Bereitstellen des Polymersubstrats das Ausbilden einer ersten im Wesentlichen flexiblen und dehnbaren Polymerfolie und das Verbinden dieser Folie mit einer zweiten im Wesentlichen flexiblen und dehnbaren Polymerfolie umfasst, die vorgedehnt wurde, oder wobei das Bereitstellen des Polymersubstrats das Verdicken eines Abschnitts des Polymersubstrats durch Aufbringen einer Lösung des im Wesentlichen flexiblen und dehnbaren Polymers auf diesen Abschnitt umfasst, vorzugsweise wobei der Schritt des Verbindens des ersten elektrischen Kontakts mit dem Polymersubstrat das Aufschleuderbeschichten einer Lösung des im Wesentlichen flexiblen und dehnbaren Polymers auf den ersten elektrischen Kontakt umfasst, um die erste im Wesentlichen flexible und dehnbare Polymerfolie zu bilden, bevor die erste im Wesentlichen flexible und dehnbare Polymerfolie mit der zweiten im Wesentlichen flexiblen und dehnbaren Polymerfolie, die vorgedehnt wurde, verbunden wird, oder worin der Schritt des Verbindens der ersten Mikronadel mit dem Polymersubstrat das Befestigen der ersten Mikronadel an dem verdickten Abschnitt des Polymersubstrats umfasst.

## Revendications

1. Dispositif portable de type transistor à effet de champ à grille étendue (EGFET) destiné à détecter et/ou à mesurer un constituant d'un liquide interstitiel à partir de la peau d'un sujet, le dispositif comprenant:
(a) un substrat polymère destiné à être fixé sur la peau du sujet;
(b) une électrode de grille étendue comprenant une première micro-aiguille configurée pour accéder au liquide interstitiel et un premier contact électrique, la première micro-aiguille et le premier contact électrique étant disposés sur le substrat polymère;
(c) une électrode de référence comprenant un deuxième contact électrique, le deuxième contact électrique étant disposé sur le substrat polymère; et
(d) un transistor à effet de champ (FET) associé à la première micro-aiguille par l'intermédiaire du premier contact électrique;
**caractérisé en ce que** l'électrode de référence comprend en outre une deuxième micro-aiguille, disposée sur le substrat polymère;
dans lequel une partie du substrat polymère sur laquelle est disposé le premier contact électrique est extensible.

2. Dispositif selon la revendication 1, dans lequel le deuxième contact électrique est disposé sur la partie extensible du substrat polymère, de préférence dans lequel la première micro-aiguille est pleine, et de manière plus préférentielle dans lequel la deuxième micro-aiguille est pleine.

3. Dispositif selon la revendication 1 ou 2, dans lequel le substrat polymère présente un gradient d'épaisseur entre la première micro-aiguille et le premier contact électrique; ou dans lequel l'épaisseur d'une partie du substrat polymère sur laquelle est disposée la première micro-aiguille est supérieure d'au moins 50% à l'épaisseur d'une partie du substrat polymère sur laquelle est disposé le premier contact électrique, de préférence dans lequel la partie du substrat polymère sur laquelle est disposée la première micro-aiguille est sensiblement rigide et la partie extensible du substrat polymère sur laquelle est disposé le premier contact électrique est souple, ou dans lequel la partie du substrat polymère sur laquelle est disposée la première micro-aiguille et la partie du substrat polymère sur laquelle est disposé le premier contact électrique sont constituées du même matériau polymère.

4. Dispositif selon l'une quelconque des revendications 1 à 3, dans lequel le substrat polymère est constitué d'un matériau polymère choisi parmi le groupe comprenant le styrène-bloc-isoprène-bloc-styrène (SIS), un copolymère à blocs 1-styrène-butadiène-styrène (SBS), un copolymère à blocs 2-styrène-éthylène-butylène-styrène (SEBS), le polydiméthylsiloxane (PDMS), le caoutchouc de polybutadiène, un élastomère thermoplastique de polyuréthane, le polyéthylène basse densité (LDPH), le polyisoprène, le caoutchouc chloroprène (CR), le caoutchouc silicone, ainsi que leurs combinaisons et dérivés; ou dans lequel la première micro-aiguille, la deuxième micro-aiguille ou les deux sont constituées d'un matériau choisi parmi le groupe comprenant un polymère, un métal, un alliage métallique, le carbone et leurs combinaisons, de préférence dans lequel le polymère est choisi parmi le groupe comprenant le polyester, le polystyrène, le polycarbonate, poly(méthacrylate de méthyle), l'acrylate, la polyvinylpyrrolidone, un photorésist négatif à base d'époxy, et leurs combinaisons, de préférence la première micro-aiguille étant constituée de polystyrène, recouverte d'un métal choisi parmi Au, Pt et Ni.

5. Dispositif selon la revendication 4, dans lequel la première micro-aiguille est modifiée par un élément de bioreconnaissance choisi parmi le groupe constitué d'une enzyme, d'un anticorps, d'un aptamère, d'une membrane sélective aux ions (ISM), d'un polymère dopé en protons, l'ADN, l'ABTS (acide 2,2'-azino-bis(3-éthylbenzothiazoline-6-sulfonique)), un polymère à empreinte moléculaire (MIP) et des combinaisons de ceux-ci, de préférence dans lequel l'élément de bioreconnaissance est lié à la première micro-aiguille par l'intermédiaire d'un lieur ou est retenu au sein d'un film ou d'une matrice de support.

6. Dispositif selon la revendication 5, dans lequel ledit constituant est un ion sodium et l'élément de bioreconnaissance comprend un ionophore Na X et du tétrakis[3,5-bis(trifluorométhyl)phényl]borate de sodium (Na-TFPB), de préférence dans lequel le matériau de bioreconnaissance est immobilisé sur la première micro-aiguille par du chlorure de polyvinyle (PVC) et de sébacate de bis(2-éthylhexyle) (DOS), de préférence dans lequel ledit constituant est le cortisol et l'élément de bioreconnaissance comprend un anticorps monoclonal anti-cortisol, et de manière encore plus préférée dans lequel l'élément de bioreconnaissance est immobilisé sur la première micro-aiguille via un lieur à base de (3-aminopropyl)triéthoxysilane (APTES) et de glutaraldéhyde; ou dans lequel ledit constituant est le glucose et l'élément de bioreconnaissance est la glucose oxydase, de préférence dans lequel l'élément de bioreconnaissance est immobilisé sur la première micro-aiguille par du chitosane, le chitosane étant mélangé à des nanotubes de carbone; ou dans lequel ledit constituant est un ion hydronium et l'élément de bioreconnaissance est la polyaniline (PANI).

7. Dispositif selon la revendication 4, dans lequel la deuxième micro-aiguille est constituée de polystyrène recouvert d'Ag, de préférence dans lequel la deuxième micro-aiguille est modifiée avec un matériau métallique configuré pour appliquer une tension à la surface de la deuxième micro-aiguille, la tension de grille du FET étant sensible au matériau métallique, plus préférentiellement dans lequel le matériau métallique est choisi parmi le groupe constitué d'Ag/AgCl/PVB et d'Au.

8. Dispositif selon l'une quelconque des revendications 1 à 7, dans lequel la première micro-aiguille, la deuxième micro-aiguille ou les deux présentent une forme conique ayant une hauteur comprise entre environ 250 µm et environ 5 mm et un diamètre à sa base compris entre environ 100 µm et 2,5 mm; ou dans lequel l'électrode de grille étendue comprend une pluralité de premières micro-aiguilles disposées sur le substrat polymère et agencées en réseau, le FET étant associé à ladite pluralité de premières micro-aiguilles par l'intermédiaire du premier contact électrique; ou dans lequel l'électrode de référence comprend une pluralité de deuxièmes micro-aiguilles disposées sur le substrat polymère et agencées en réseau; ou dans lequel le premier contact électrique, le deuxième contact électrique ou les deux comprennent des nanostructures allongées électriquement conductrices, de préférence dans lequel les nanostructures allongées électriquement conductrices sont choisies parmi le groupe constitué de nanotubes, de nanofils, de nanorubans, des nano-poils, des nano-bandes, des nanobâtonnets et des combinaisons de ceux-ci, de préférence en ce que les nanostructures allongées électriquement conductrices sont constituées d'un matériau choisi parmi le groupe comprenant un métal, un alliage métallique, le carbone et des combinaisons de ceux-ci, et de manière encore plus préférentielle en ce que le premier contact électrique et le deuxième contact électrique comprennent des nanofils d'argent (AgNWs).

9. Dispositif selon l'une quelconque des revendications 1 à 8, dans lequel le FET est choisi parmi le groupe constitué d'un transistor à effet de champ métal-oxyde-semi-conducteur (MOSFET), d'un transistor à nanofils sans jonction (JLNT), d'un transistor métal-nitrure-oxyde-semi-conducteur (MNOS), d'un transistor à effet de champ à jonction (JFET), d'un transistor à induction statique (SIT); un transistor à effet de champ à grille isolée à hétérostructure (HIGFET), un transistor à effet de champ à dopage de modulation (MODFET); transistor à effet de champ à effet tunnel (TFET), transistor à haute mobilité électronique (HEMT), transistor à effet de champ métal-semi-conducteur (MESFET), transistor à effet de champ à mémoire organique à nanoparticules (NOMFET), transistor à effet de champ à nanorubans de graphène (GNRFET), transistor à effet de champ à fente verticale (VeSFET), transistor à effet de champ à nanotubes de carbone (CNTFET), transistor à effet de champ organique (OFET), transistor à effet de champ quantique (QFET), transistor à effet de champ à barrière de Schottky (SB-FET) et transistor à effet de champ à base de graphène (GFET), de préférence dans lequel le FET est un MOSFET.

10. Système destiné à détecter et/ou à mesurer un constituant d'un liquide interstitiel provenant de la peau d'un sujet, le système comprenant:
le dispositif EGFET portable selon l'une quelconque des revendications 1 à 9; et
au moins l'un des éléments suivants:
une unité de commande en communication électrique avec le dispositif EGFET, qui mesure un signal électrique généré par le FET en réponse à une interaction entre la première micro-aiguille et ledit constituant; et
un émetteur, qui reçoit le signal électrique généré par le FET en réponse à l'interaction entre la première micro-aiguille et ledit constituant et transmet ledit signal à un serveur distant et/ou à un dispositif électronique portable.

11. Système selon la revendication 10, dans lequel l'unité de commande est en communication électrique avec chacun des éléments suivants: le FET, l'électrode de grille étendue et l'électrode de référence au sein du dispositif EGFET; ou dans lequel le système comprend en outre une unité d'affichage en communication électrique avec l'unité de commande pour afficher des informations relatives à la mesure du signal électrique, et/ou des moyens pour déterminer la concentration du constituant dans le liquide interstitiel à la réception du signal électrique.

12. Procédé de détection et/ou de mesure d'un constituant d'un liquide interstitiel à partir de la peau d'un sujet, le procédé comprenant:
(a) fournir le système selon l'une quelconque des revendications 10 à 11;
(b) fixer l'EGFET portable sur la peau d'un sujet, permettant ainsi une interaction entre la première micro-aiguille et ledit constituant;
(c) mesurer un signal électrique généré par le FET en réponse à l'interaction entre la première micro-aiguille et ledit constituant; et
(d) analyser le signal électrique par au moins l'un parmi l'unité de commande, le serveur distant et le dispositif électronique portable.

13. Procédé selon la revendication 12, dans lequel fixer l'EGFET portable sur la peau du sujet consiste à mettre la première micro-aiguille en contact direct avec la peau du sujet, et dans lequel l'étape de mesure est réalisée pendant que l'EGFET portable est fixé sur la peau du sujet; ou dans lequel analyser le signal électrique consiste à comparer le signal électrique à une courbe d'étalonnage et/ou à des données de référence; ou dans laquelle le procédé comprend en outre l'affichage d'informations relatives à la mesure du signal électrique ou d'un résultat de l'étape d'analyser (étape (d)) sur une unité d'affichage en communication électrique avec l'unité de commande, le serveur distant ou le dispositif électronique portable.

14. Procédé de fabrication du dispositif EGFET portable selon l'une quelconque des revendications 1 à 9, le procédé comprenant:
(a) fournir la première micro-aiguille, le premier contact électrique et le substrat polymère;
(b) fournir le FET;
(c) connecter le premier contact électrique au substrat polymère;
(d) connecter la première micro-aiguille au substrat polymère; et
(e) associer le FET à la première micro-aiguille.

15. Procédé selon la revendication 14, dans lequel les étapes (a) et (b) sont réalisées dans n'importe quel ordre ou dans lequel les étapes (a) et (b) sont réalisées simultanément, ou dans lequel le procédé comprend en outre une étape consistant à fournir la deuxième micro-aiguille et le deuxième contact électrique, et une étape consistant à connecter la deuxième micro-aiguille et le deuxième contact électrique au substrat polymère, de préférence dans lequel fournir le premier contact électrique et/ou fournir le deuxième contact électrique comprend le revêtement par pulvérisation de nanostructures allongées hydrophobes et électriquement conductrices sur une plaquette de silicium recouverte d'un masque présentant une ouverture prédéfinie, le retrait du masque et le recuit du premier contact électrique et/ou du deuxième contact électrique obtenus, ou dans lequel fournir le substrat polymère comprend la formation d'un premier film polymère sensiblement souple et extensible et la combinaison dudit film avec un deuxième film polymère sensiblement souple et extensible, qui a été pré-étiré, ou dans lequel fournir le substrat polymère comprend l'épaississement d'une partie du substrat polymère par application d'une solution du polymère sensiblement souple et extensible sur ladite partie, de préférence, dans lequel l'étape consistant à connecter le premier contact électrique au substrat polymère comprend l'application par centrifugation d'une solution du polymère sensiblement souple et extensible sur le premier contact électrique afin de former le premier film polymère sensiblement souple et extensible avant d'associer ledit premier film polymère sensiblement souple et extensible au deuxième film polymère sensiblement souple et extensible, qui a été pré-étiré, ou dans lequel l'étape consistant à connecter la première micro-aiguille au substrat polymère comprend fixer la première micro-aiguille sur la partie épaissie du substrat polymère.
